(19) Europäisches Patentamt
European Patent Office
Office européen des brevets

(11) EP 1 490 409 B1

(12) **EUROPEAN PATENT SPECIFICATION**

(45) Date of publication and mention of the grant of the patent:
**31.12.2008 Bulletin 2009/01**

(21) Application number: **03712543.2**

(22) Date of filing: **26.03.2003**

(51) Int Cl.:
***C08B 37/00*** *(2006.01)* ***A61K 39/385*** *(2006.01)*
***A61K 39/095*** *(2006.01)*

(86) International application number:
**PCT/IB2003/001436**

(87) International publication number:
**WO 2003/080678 (02.10.2003 Gazette 2003/40)**

(54) **MODIFIED SACCHARIDES HAVING IMPROVED STABILITY IN WATER**

MODIFIZIERTE SACCHARIDE MIT VERBESSERTER STABILITÄT IN WASSER

SACCHARIDES MODIFIES PRESENTANT UNE STABILITE AMELIOREE DANS L'EAU

(84) Designated Contracting States:
**AT BE BG CH CY CZ DE DK EE ES FI FR GB GR HU IE IT LI LU MC NL PT RO SE SI SK TR**

(30) Priority: **26.03.2002 GB 0207117**
**30.08.2002 GB 0220195**
**18.12.2002 GB 0229494**
**24.12.2002 GB 0230163**

(43) Date of publication of application:
**29.12.2004 Bulletin 2004/53**

(60) Divisional application:
**06076853.8 / 1 777 236**

(73) Proprietor: **Novartis Vaccines and Diagnostics S.r.l.**
**53100 Siena (SI) (IT)**

(72) Inventors:
- **COSTANTINO, Paolo, Chiron S.r.l. I-53100 Siena (IT)**
- **BERTI, Francesco, Chiron S.r.l. I-53100 Siena (IT)**
- **NORELLI, Francesco, Chiron S.r.l. I-53100 Siena (IT)**
- **BARTOLONI, Antonella, Chiron S.r.l. I-53100 Siena (IT)**

(74) Representative: **Marshall, Cameron John et al**
**Carpmaels & Ransford**
**43-45 Bloomsbury Square**
**London WC1A 2RA (GB)**

(56) References cited:
**WO-A-93/07178**
**WO-A-98/08543**
**WO-A-98/42718**
**WO-A-99/32653**
**WO-A-03/007985**
**GB-A- 733 459**
**GB-A- 2 009 198**
**US-A- 4 727 136**
**US-A- 4 882 317**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

This invention is in the field of polysaccharide chemistry and relates to modified saccharides, processes for their preparation, and conjugated derivatives. In particular, the invention relates to modified saccharides having improved stability in water.

### BACKGROUND ART

Polysaccharides are important biological molecules and they have been widely used in the pharmaceutical industry for the prevention and treatment of diseases. For example, capsular polysaccharides have been used for many years in vaccines against capsulated bacteria, such as meningococcus (*Neisseria meningitidis*), pneumococcus (*Streptococcus pneumoniae*) and Hib *(Haemophilus influenzae* type B).

To enhance immunogenicity of these polysaccharides, particularly in children, conjugate vaccines were developed. These comprise a capsular polysaccharide conjugated to a carrier protein [*e.g*. references 1, 2, 3]. Conjugation can make T-independent antigens into T-dependent antigens.

A problem with many types of polysaccharide is poor stability in water. The stability of polysaccharides in water depends on the nature of the O-glycosidic bonds joining the saccharide units. Poor stability in water is a result of the O-glycosidic bonds being readily hydrolysed in the presence of acids or glycosidases. The capsular polysaccharide of serogroup A meningococcus is an example of a polysaccharide having poor stability in water.

The stability of polysaccharides is a particular problem in the manufacture of conjugate vaccines. In order to prepare a polysaccharide-protein conjugate, it is necessary to manipulate chemically functional groups on the polysaccharide so that the polysaccharide may be linked to a protein. The polysaccharide may be linked either directly to the protein [2, 4] or it may be linked via a linker group. Many different types of linker groups have been proposed for linking polysaccharides to proteins [*e.g*. 3, 5].

The exposure of a polysaccharide to chemical reagents, particularly acids, may result in undesirable cleavage of glycosidic linkages and consequent fragmentation of the polysaccharide. Such fragmentation is highly undesirable, causing loss in yields in the synthesis of polysaccharide-protein conjugates.

Polysaccharides which are unstable in this way generally require careful choice of reagents and conditions to circumvent the problems described above. However, this limits the reagents available for manipulating the polysaccharide, thus limiting the range of linkages which may be made between the polysaccharide and carrier protein. In addition, the instability of these polysaccharides means it is difficult to develop robust procedures, which can be used to prepare vaccines on an industrial scale.

It is an object of the invention to provide ways of modifying capsular saccharides so that they do not suffer from instability problems and maintain immunogenicity.

GB733459 discloses pyrogenic polysaccharides derived from gram negative bacteria that are acylated, e.g. with an organic acid anhydride. The products are stated to be useful as stimulating agents for the pituitary adrenocortical system with reduced side-effects.

WO 98/42718 discloses a method for depolymerising polysaccharides into saccharide fragments using ozonolysis.

WO 93/07178 discloses an oligosaccharide derived from an antigenic polysaccharide obtained from a pathogenic agent, a method for its preparation, and its use particularly as a vaccine. The oligosaccharide is prepared by an oxidation-reduction depolymerisation reaction.

### DISCLOSURE OF THE INVENTION

The invention is based on the discovery that modification of hydroxyl groups on monosaccharide units of capsular saccharides offers improved stability. Modified saccharides obtained by the process of the invention are more stable to hydrolysis than their native saccharide counterparts.

#### *Modified saccharides of the invention*

The invention provides a modified capsular saccharide comprising a blocking group at a hydroxyl group position on at least one of the monosaccharide units of the corresponding native capsular saccharide, wherein the capsular saccharide comprises phosphodiester linkages.

The term "modified capsular saccharide" means a saccharide which is obtainable from a native capsular saccharide by suitable modification. Hence, the basic sequence of repeating monosaccharide units in the native capsular

saccharide is retained in the modified capsular saccharides of the present invention.

The term "saccharide" encompasses both oligosaccharides (*e.g*. containing from 2 to 39 monosaccharide units) and polysaccharides (*e.g*. containing 40 or more monosaccharide units). As found naturally in bacteria, native capsular saccharides generally take the form of polysaccharides. Polysaccharides may be manipulated to give shorter oligosaccharides. Oligosaccharides may be obtained by purification and/or sizing of the native polysaccharide (*e.g*. by hydrolysis in mild acid, by heating, by sizing chromatography *etc*.).

Typically, the modified saccharides of the present invention are oligosaccharides. Oligo-saccharides may be obtained from polysaccharides by any of the sizing methods described above.

The modified capsular saccharides of this invention are obtainable from native capsular saccharides. However, the present invention is not limited to modified saccharides obtained from native capsular saccharides. The modified capsular saccharides of the present invention may be obtained by other methods, such as total or partial synthesis.

The number of monosaccharide units having blocking groups may vary in the present invention. For example, all or substantially all the monosaccharide units of the corresponding capsular saccharide may have blocking groups. Alternatively, at least 10%, 20%, 30%, 40%, 50%, 60%, 70%, 80% or 90% of the monosaccharide units of the corresponding capsular saccharide may have blocking groups. At least 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 monosaccharide units of the corresponding capsular saccharide may have blocking groups.

Likewise, the number of blocking groups on a monosaccharide unit may vary. For example, the number of blocking groups on a monosaccharide unit may be 1, 2, 3, 4, 5 or 6, preferably 1-4, more preferably 1-2.

In one embodiment, the at least one monosaccharide unit having a blocking group is a non-terminal monosaccharide unit. The term "non-terminal monosaccharide unit" means a monosaccharide unit which is not one of the terminal monosaccharide units in the oligosaccharide/polysaccharide chain.

This invention encompasses modified capsular saccharides wherein all the hydroxyl group positions of the terminal and non-terminal monosaccharide units have a blocking group. However, it is preferred that there is at least one free hydroxyl group or amino group in the modified capsular saccharide of the present invention. A free hydroxyl group or amino group is advantageous because it provides a handle for further reactions of the modified capsular saccharide *e.g*. for conjugation to a carrier molecule. When the modified saccharide contains a free hydroxyl group, it is preferably an anomeric hydroxyl group, preferably a terminal anomeric hydroxyl group. When the modified saccharide contains an amino group, it is preferably derived from an anomeric hydroxyl group. Amino groups are readily accessible from anomeric hydroxyl groups by reductive amination (using, for example, $NaBH_3CN/NH_4Cl$).

The term "amino group" includes groups of the formula $-NH_2$ or NH-E, where E is a nitrogen protecting group. Examples of typical nitrogen protecting groups are described below.

The term "blocking group" means any group which blocks the reactivity of a hydroxyl group. The skilled person will be aware of many different types of blocking group. Preferred blocking groups for hydroxyl groups are groups which are directly accessible via a derivatizing reaction of the hydroxyl group *i.e*. by replacing the hydrogen atom of the hydroxyl group with another group. Suitable derivatives of hydroxyl groups which act as blocking groups are, for example, carbamates, sulfonates, carbonates, esters, ethers (*e.g*. silyl ethers or alkyl ethers) and acetals. Some specific examples of such blocking groups are allyl, Aloc, benzyl, BOM, t-butyl, trityl, TBS, TBDPS, TES, TMS, TIPS, PMB, MEM, MOM, MTM, and THP.

However, the blocking group need not be directly accessible via a derivatizing reaction of the hydroxyl group. The blocking group may completely replace the hydroxyl group. For example, the blocking group may be $C_{1-12}$ alkyl, $C_{3-12}$ alkyl, $C_{5-12}$ aryl, $C_{5-12}$ aryl-$C_{1-6}$ alkyl, $NR^1R^2$ (where $R^1$ and $R^2$ are as defined below), H, F, Cl, Br, $CO_2H$, $CO_2(C_{1-6}$ alkyl), CN, $CF_3$, $CCl_3$ *etc.*

Preferably, the blocking group is an electron-withdrawing group. Without wishing to be bound by theory, it is believed that glycosidic bonds are unstable to hydrolysis due to assistance from an intramolecular nucleophilic attack of a saccharide hydroxyl group on the glycosidic linkage (*i.e*. by formation of a cyclic intermediate). The greater the nucleophilicity of the hydroxyl group, the greater the tendency for intramolecular nucleophilic attack. An electron-withdrawing blocking group has the effect of delocalizing the oxygen lone pair, thereby decreasing the oxygen nucleophilicity and decreasing the tendency for intramolecular nucleophilic attack.

Preferably, the blocking group is of the formula:

$$-O-X-Y \text{ or } -OR^3$$

wherein

X is C(O), S(O) or $SO_2$;

Y is $C_{1-12}$ alkyl, $C_{1-12}$ alkoxy, $C_{3-12}$ cycloalkyl, $C_{5-12}$ aryl or $C_{5-12}$ aryl-$C_{1-6}$ alkyl, each of which may optionally be substituted with 1, 2 or 3 groups independently selected from F, Cl, Br, $CO_2H$, $CO_2(C_{1-6}$ alkyl), CN, $CF_3$ or $CCl_3$; or Y is $NR^1R^2$;

$R^1$ and $R^2$ are independently selected from H, $C_{1-12}$ alkyl, $C_{3-12}$ cycloalkyl, $C_{5-12}$ aryl, $C_{5-12}$ aryl-$C_{1-6}$ alkyl; or $R^1$ and $R^2$ may be joined to form a $C_{3-12}$ saturated heterocyclic group;

$R^3$ is $C_{1-12}$ alkyl or $C_{3-12}$ cycloalkyl, each of which may optionally be substituted with 1, 2 or 3 groups independently selected from F, Cl, Br, $CO_2$($C_{1-6}$ alkyl), CN, $CF_3$ or $CCl_3$; or $R^3$ is $C_{5-12}$ aryl or $C_{5-12}$ aryl-$C_{1-6}$ alkyl, each of which may optionally be substituted with 1, 2, 3, 4 or 5 groups selected from F, Cl, Br, $CO_2H$, $CO_2$($C_{1-6}$ alkyl), CN, $CF_3$ or $CCl_3$;

Preferably, when $R^3$ is $C_{1-12}$ alkyl or $C_{3-12}$ cycloalkyl, it is substituted with 1, 2 or 3 groups as defined above.

The blocking groups of formula -O-X-Y or $-OR^3$ may be prepared from hydroxyl groups by standard derivatizing procedures, such as reaction of the hydroxyl group with an acyl halide, alkyl halide, sulfonyl halide *etc*. Hence, the oxygen atom in -O-X-Y is preferably the oxygen atom of the hydroxyl group, while the -X-Y group in -O-X-Y preferably replaces the hydrogen atom of the hydroxyl group.

Alternatively, the blocking groups may be accessible via a substitution reaction, such as a Mitsonobu-type substitution. These and other methods of preparing blocking groups from hydroxyl groups are well known.

More preferably, the blocking group is $-OC(O)CF_3$ [6] or $-OC(O)NR^1R^2$.

More preferably, the blocking group is a carbamate group of the formula $-OC(O)NR^1R^2$, wherein $R^1$ and $R^2$ are independently selected from $C_{1-6}$ alkyl. More preferably, $R^1$ and $R^2$ are both methyl *i.e.* the blocking group is -OC (O)$NMe_2$.

Carbamate blocking groups have a stabilizing effect on the glycosidic bond and may be prepared under mild conditions. An example of a process for manipulating a saccharide to provide a carbamate blocking group is described below. However, the invention is not limited to modified saccharides prepared by the processes exemplified herein, and other processes for preparing modified saccharides of the invention will be readily apparent to the skilled person.

The term "alkyl" is used herein to refer to alkyl groups in both straight and branched forms, The alkyl group may be interrupted with 1, 2 or 3 heteroatoms selected from -O-, -NH- or -S-. The alkyl group may also be interrupted with 1, 2 or 3 double and/or triple bonds. However, the term "alkyl" usually refers to alkyl groups having no heteroatom interruptions or double or triple bond interruptions. Where reference is made to $C_{1-12}$ alkyl, it is meant the alkyl group may contain any number of carbon atoms between 1 and 12 (*e.g.* $C_1$, $C_2$, $C_3$, $C_4$, $C_5$, $C_6$, $C_7$, $C_8$, $C_9$, $C_{10}$, $C_{11}$, $C_{12}$). Similarly, where reference is made to $C_{1-6}$ alkyl, it is meant the alkyl group may contain any number of carbon atoms between 1 and 6 (*e.g.* $C_1$, $C_2$, $C_3$, $C_4$, $C_5$, $C_6$).

The term "cycloalkyl" includes cycloalkyl, polycycloalkyl, and cycloalkenyl groups, as well as combinations of these with alkyl groups, such as cycloalkylalkyl groups. The cycloalkyl group may be interrupted with 1, 2 or 3 heteroatoms selected from -O-, -NH- or -S-. However, the term "cycloalkyl" usually refers to cycloalkyl groups having no heteroatom interruptions Examples of cycloalkyl groups include cyclopentyl, cyclohexyl, cyclohexenyl, cyclohexylmethyl and adamantyl groups. Where reference is made to $C_{3-12}$ cycloalkyl, it is meant that the cycloalkyl group may contain any number of carbon atoms between 3 and 12 (*e.g.* $C_3$, $C_4$, $C_5$, $C_6$, $C_7$, $C_8$, $C_9$, $C_{10}$, $C_{11}$, $C_{12}$).

The term "aryl" is used herein to refer to an aromatic group, such as phenyl or naphthyl. Where reference is made to $C_{5-12}$ aryl, it is meant that the aryl group may contain any number of carbon atoms between 5 and 12 (*e.g.* $C_5$, $C_6$, $C_7$, $C_8$, $C_9$, $C_{10}$, $C_{11}$, $C_{12}$).

The term "$C_{5-12}$ aryl-$C_{1-6}$ alkyl" refers to groups such as benzyl, phenylethyl and naphthylmethyl.

When $R^1$ and $R^2$ are joined to form a $C_{3-12}$ saturated heterocyclic group, it is meant that $R^1$ and $R^2$ together with the nitrogen atom form a saturated heterocyclic group containing any number of carbon atoms between 3 and 12 (*e.g.* $C_3$, $C_4$, $C_5$, $C_6$, $C_7$, $C_8$, $C_9$, $C_{10}$, $C_{11}$, $C_{12}$). The heterocyclic group may contain 1 or 2 heteroatoms (such as N, O or S) other than the nitrogen atom. Examples of $C_{3-12}$ saturated heterocyclic groups are pyrrolidinyl, piperidinyl, morpholinyl, piperazinyl, imidazolidinyl, azetidinyl and aziridinyl.

In all the embodiments described above, the modified capsular saccharide is a modified capsular saccharide having phosphodiester linkages. More preferably, the modified capsular saccharide is a modified *Neisseria meningitidis* serogroup A saccharide. *Neisseria meningitidis* serogroup A saccharides are particularly unstable to hydrolysis.

When the modified capsular saccharide is a modified *Neisseria meningitidis* serogroup A saccharide, the blocking group is preferably at the 4 and/or 3-positions, more preferably the 4-position, of the corresponding *Neisseria meningitidis* serogroup A saccharide. Blocking groups in the 4 and/or 3-positions *Neisseria meningitidis* serogroup A saccharide have been shown to be particularly efficacious for improving stability towards hydrolysis.

This invention also provides a saccharide of the formula:

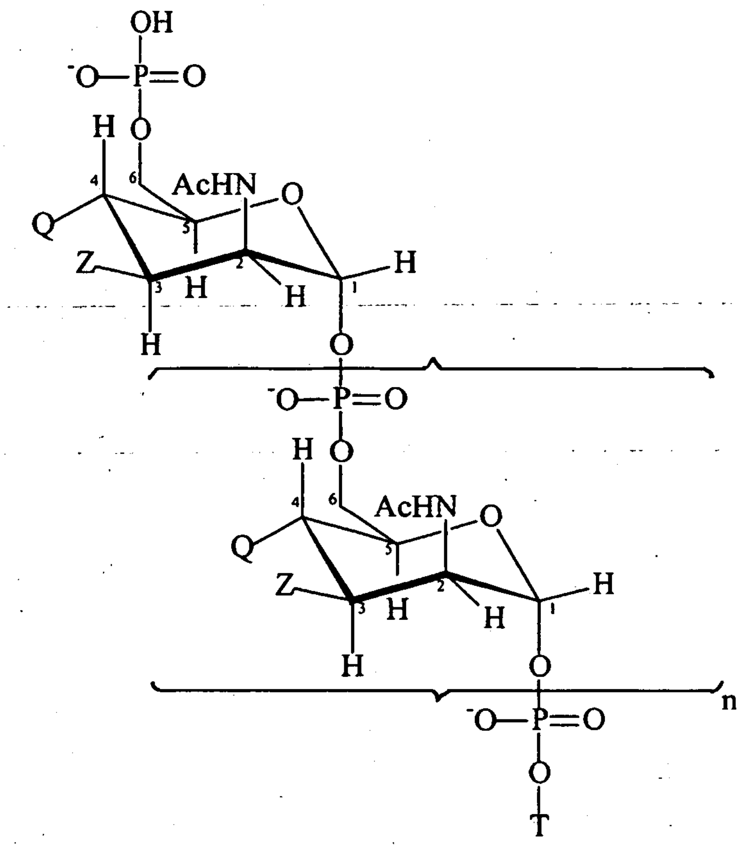

wherein

T is of the formula (A) or (B):

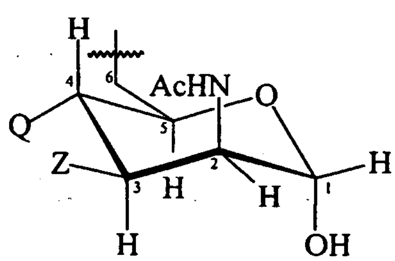

(A)

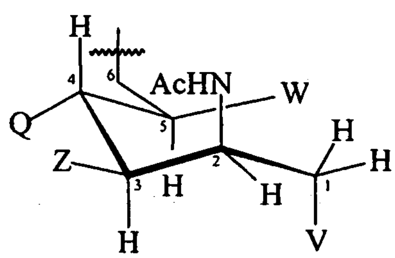

(B)

n is an integer from 1 to 100;

each Z group is independently selected from -OH or a blocking group as defined above; and

each Q group is independently selected from -OH or a blocking group as defined above;

W is selected from -OH or a blocking group as defined above;

V is selected from $-NH_2$, -NHE, $-NE^1E^2$, -OH, or -O-D, where: E, $E^1$ and $E^2$ are nitrogen protecting groups, which may be the same or different, and D is an oxygen protecting group.

and wherein more than about 7% *(e.g.* 8%, 9%, 10% or more) of the Q groups are blocking groups.

Preferably, n is an integer from 15 to 25.

Each of the n+2 Z groups may be the same or different from each other. Likewise, each of the n+2 Q groups may be the same or different from each other.

V is preferably $-NH_2$ or -NHE.

Suitable nitrogen protecting groups are silyl groups (such as TMS, TES, TBS, TIPS), acyl derivatives (such as trifluoroacetamides, methoxycarbonyl, ethoxycarbonyl, t-butoxycarbonyl (Boc), benzyloxycarbonyl (Z or Cbz), 9-fluorenylmethoxycarbonyl (Fmoc), 2-(trimethylsilyl)ethoxy carbonyl, allyloxycarbonyl (Alloc), 2,2,2-trichloroethoxycarbonyl

(Troc)), sulfonyl derivatives (such as â-trimethylsilylethanesulfonyl (SES)), sulfenyl derivatives, $C_{1-12}$ alkyl, benzyl, benzhydryl, trityl, allyl, 9-phenylfluorenyl, *etc.* A preferred nitrogen protecting group is Fmoc.

Divalent nitrogen protecting groups, which can be used as $E^1E^2$, include cyclic imide derivatives (such as N-phthalimides, N-dithiasuccinimides, N-2,3-diphenylmaleimides), imine derivatives (such as N-1,1-dimethylthiomethyleneamines, N-benzylideneamines, N-p-methoxybenzylideneamines, N-diphenylmethyleneamines), enamine derivatives (such as N-(5,5-dimethyl-3-oxo-1-cyclohexenyl)amines), *etc.* A preferred divalent nitrogen protecting group is N-phthalimidyl.

Suitable oxygen protecting groups include esters, ethers (e.g. silyl ethers or alkyl ethers) and acetals. Specific examples include allyl, acetyl, Aloc, benzyl, benzyloxymethyl (BOM), t-butyl, trityl, tertbutyldimethylsilyl (TBS), tert-butyldiphenylsilyl (TBDPS), triethylsilyl (TES), trimethylsilyl (TMS), tri-isopropylsilyl (TIPS), paramethoxybenzyl (PMB), MEM, methoxymethyl (MOM), MTM and tetrahydropyranyl (THP).

All the Z groups may be OH. Alternatively, at least 10%, 20, 30%, 40%, 50% or 60% of the Z groups may be OAc. Preferably, about 70% of the Z groups are OAc, with the remainder of the Z groups being OH or blocking groups as defined above.

At least about 7% of Q groups are blocking groups. Preferably, at least 10%, 20%, 30%, 40%, 50%, 60%, 70%, 80% or 90% of the Q groups are blocking groups. Alternatively, all the Q groups may be blocking groups.

The invention also provides a molecule comprising a saccharide moiety of formula:

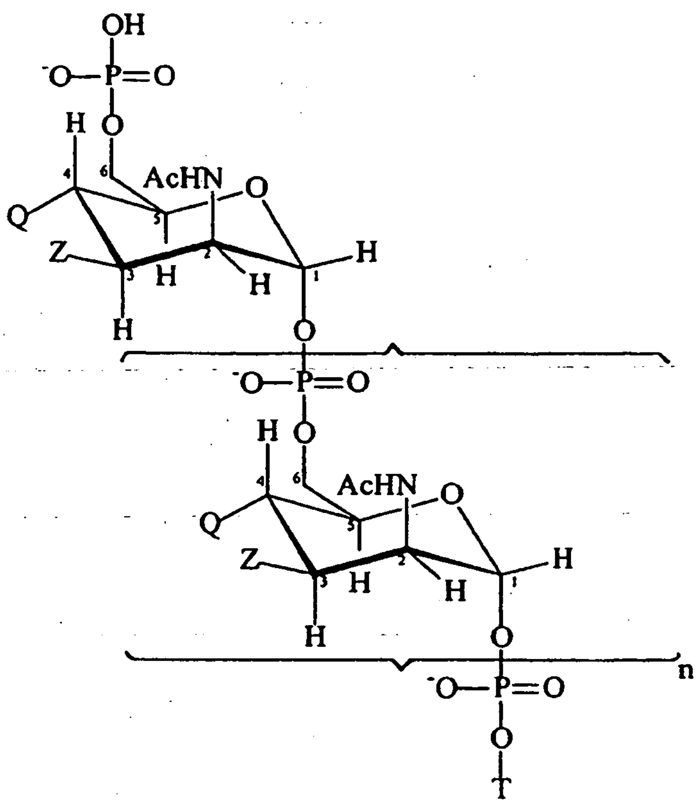

wherein
T is of the formula (A) or (B):

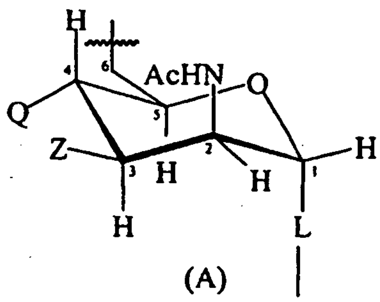

(A)

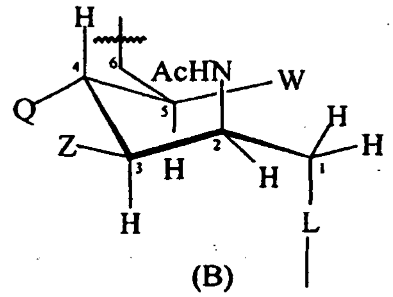

(B)

n, Z, Q and W are as defined above, and: L is O, NH, NE, S or Se.

The free covalent bond of L can be joined to any appropriate moiety e.g. to -H, -E, a linker, a protein carrier, *etc*. L is preferably N or O. It is also possible for L to be N, joined to a divalent linker, to a divalent protecting group, or to a divalent protein carrier.

***Process for producing a modified saccharide***

The invention provides a process for modifying a capsular saccharide comprising the steps of:

(a) providing a capsular saccharide having at least one hydroxyl group on a monosaccharide unit; and
(b) converting said at least one hydroxyl group into a blocking group; wherein the capsular saccharide comprises phosphodiester linkages.

The blocking group may be any of the blocking groups defined above.

The capsular saccharide may be a native capsular saccharide (oligosaccharide or polysaccharide). As an alternative, the capsular saccharide may be, for example, a de-O-acetylated capsular saccharide and/or a capsular saccharide having a terminal amino group (*e.g.* obtained by reductive amination).

A preferred process for modifying a saccharide wherein the blocking group is $-OC(Q)NR^1R^2$ is when step (b) comprises the steps of:

(b1) reacting the capsular saccharide with a bifunctional reagent in an organic solvent; and
(b2) reacting the product of step (b1) with an amino compound of formula (I):

$$HNR^1R^2 \qquad (I)$$

wherein $R^1$ and $R^2$ are as defined above, and the capsular saccharide comprises phosphodiester linkages.

The term "bifunctional reagent" means any reagent which is capable of performing the dual functions of (i) providing in step (b1) a first electrophilic carbon atom for coupling with the hydroxyl group(s) on the saccharide; and (ii) providing a second electrophilic carbon atom for coupling with the amino group used in step (b2). Generally, the second electrophilic carbon atom is regenerated from the first electrophilic carbon atom during step (b). The bifunctional reagent provides a -C(O)- linkage between the polysaccharide and the amino compound.

Bifunctional reagents for use in the present invention include, but are not limited to, 1,1'-carbonyldiimidazole (CDI), carbonyl di-1,2,4-triazole (CDT), carbonyl di-1,2,3-benzotriazole (CDB), diphenylcarbonate, cyanogen bromide, phosgene or triphosgene. The skilled person will be aware of other bifunctional reagents which can perform the same function as these.

A preferred bifunctional reagent is CDI. CDI has the advantage of being a milder reagent than, for example, phosgene or cyanogen bromide. In particular, coupling reactions using CDI do not generate hydrohalic acid gases, such as HCl or HBr. The generation of HCl or HBr gas is undesirable, because these gases require scrubbing of the reaction chamber outlet to avoid their escape into the atmosphere. Moreover, the generation of HCl or HBr gas may affect sensitive functional groups on the saccharide, resulting in loss in yields due to decomposition or fragmentation of the saccharide.

The organic solvent used in step (b1) is preferably an aprotic solvent. Aprotic solvents are well known to the person skilled in the art and do not contain any ionizable hydrogen atoms. These solvents are advantageous because they facilitate the reaction of hydroxyl group(s) on the saccharide with the bifunctional reagent, by enhancing the nucleophilicity of the hydroxyl group(s). Suitable aprotic solvents include, but are not limited to, dimethylsulfoxide (DMSO),

dimethylformamide (DMF), formamide, hexamethylphosphorus triamide (HMPT), 1,3-dimethyl-3,4,5,6-tetrahydro-2(1*H*)-pyrimidinone (DMPU), dimethylacetamide (DMAC), or hexamethylphosphoramide (HMPA). DMSO is preferred.

In step (b2) of the process of the invention, the product of step (b1) is reacted with an amino compound to form the modified polysaccharide. The amino compound used in the process of the present invention is of formula (I), as defined above. In formula (I), preferably, $R^1$ and $R^2$ are independently selected from $C_{1-6}$ alkyl. More preferably $R^1$ and $R^2$ are both methyl.

Suitable amino compounds which may be used in the invention are methylamine, dimethylamine, ethylamine, *N*-ethylmethylamine, diethylamine, *N*-methylpropylamine, *N*-ethylpropylamine, isopropylamine, butylamine, *N*-methylbutylamine, *N*-ethylbutylamine, *N*-propylbutylamine, *N*-methylcyclopentylamine, *N*-ethylcyclopentylamine, cyclohexylamine, *N*-methylcyclohexylamine, *N*-ethylcyclohexylamine, benzylamine, *N*-ethylbenzylamine, *N*-methylbenzylamine, isobutylamine, tert-butylamine, cyclopentylamine, dibenzylamine, pyrrolidine, piperidine, morpholine, piperazine, imidazolidine, azetidine, aziridine, aniline, *N*-methylaniline and *N*-ethylaniline. These may be used in the salt form (*e.g.* hydrochloride salt).

Preferably, the amino compound used in the present invention is a secondary amine. More preferably, the amine is dimethylamine.

A preferred process of the invention is exemplified in Scheme 1 below:

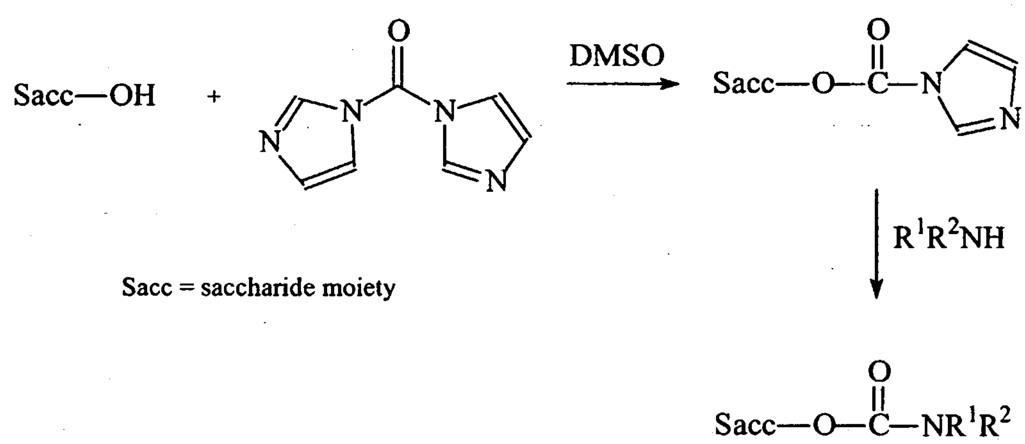


**Scheme 1**

In this scheme, the saccharide (*e.g.* MenA polysaccharide or oligosaccharide) is first activated through at least one of its hydroxyl groups on a monosaccharide unit using CDI in DMSO solvent. The resulting imidazole carbamate intermediate is trapped by the amine $R^1R^2NH$ (*e.g.* dimethylamine) to give the modified saccharide.

The modified saccharides may alternatively be prepared in a one-step process by reacting one or more hydroxyl groups on a capsular saccharide with a reagent of the formula $XC(O)NR^1R^2$, wherein X is a leaving group, and $R^1$ and $R^2$ are as defined above. Suitable leaving groups include, but are not limited to, -Cl, -Br, $-CF_3$, $-OC_6F_5$ or $-CCl_3$.

Alternatively, modified capsular saccharides of the present invention may be prepared by synthetic means, for example, from suitable monosaccharide units. Typically, total synthesis of a modified capsular saccharide comprises forming glycosidic linkages (*e.g.* phosphodiester linkages) between suitable monosaccharide units and then modifying the resultant saccharide in any manner described above. Alternatively, the monosaccharide units may be modified before forming the glycosidic linkages to provide the same modified capsular saccharide.

The modified capsular saccharides of this invention are preferably oligosaccharides. Starting from native capsular polysaccharides, modified capsular oligosaccharides may be obtained by either of two methods: (1) modifying the capsular polysaccharide followed by sizing the modified polysaccharide to form a modified oligosaccharide; or (2) sizing the capsular polysaccharide followed by modifying the resultant oligosaccharide to form a modified oligosaccharide. Both methods are encompassed within the present invention. However, the first method is preferred, since this method ensures that a terminal hydroxyl group will be available for subsequent conjugation of the modified oligosaccharide to a carrier molecule, such as a protein.

The present invention also provides a process for modifying a *Neisseria meningitidis* serogroup A polysaccharide comprising the steps of:

(a) providing a *Neisseria meningitidis* serogroup A polysaccharide;

(b) sizing said polysaccharide to provide an oligosaccharide; and
(c) converting at least one hydroxyl group of the oligosaccharide into a blocking group, as described above.

Step (b) of this process may optionally be followed by known derivatizing step(s) before step (c). Known derivatizing steps include, for example, reductive amination followed by protection of the resulting -$NH_2$ group and/or de-O-acetylation.
This invention also provides a process for modifying a *Neisseria meningitidis* serogroup A polysaccharide comprising the steps of:

(a) providing a *Neisseria meningitidis* serogroup A polysaccharide;
(b) converting at least one hydroxyl group of the polysaccharide into a blocking group, as described above; and
(c) sizing the resulting polysaccharide to provide an oligosaccharide.

Step (c) of this process may optionally be followed by known derivatizing step(s). Known derivatizing steps include, for example, reductive amination followed by protection of the resulting -$NH_2$ group and/or de-O-acetylation.
Any of the processes described above may be followed by a step in which contaminants *(e.g.* low molecular weight contaminants) are removed.

***Capsular saccharide starting materials***

The modified capsular saccharides of the invention are obtainable from native capsular saccharides. The term "native capsular saccharide" refers to sugar-containing polymers (e.g. polymers of sugars, sugar acids, amino sugars, polyhydric alcohols, sugar alcohols, and sugar phosphates *etc*.) which may be found in the capsule of bacteria (both Gram-positive and Gram-negative) such as *N.meningitidis, S.pneumoniae* and *H.influenzae*. Furthermore, "native capsular saccharide" includes both polysaccharides and oligosaccharides. Native capsular oligosaccharides may be obtained by sizing native polysaccharides.
The "hydroxyl group position" of a native capsular saccharide is a position on the native capsular saccharide having a hydroxyl group. However, it does not include positions in glycosidic linkages, or the residues thereof, having hydroxyl groups (*e.g*. a hydroxyl group which is part of a phosphate linkage does not occupy a hydroxyl group position). Nor does it include positions occupied by an anomeric hydroxyl group on a terminal monosaccharide unit. Positions where there is an acetoxy group (AcO) group on the native capsular saccharide are also not hydroxyl group positions.
The native capsular saccharide comprises saccharide units linked by phosphodiester bonds. Saccharides comprising phosphodiester bonds are unstable to hydrolysis.
The native capsular saccharide and the modified capsular saccharide of the invention are preferably immunogenic in mammals (*e.g*. humans). The mammal may be a human adult or a child.
The native capsular saccharide is preferably a polysaccharide (or oligosaccharide fragment thereof) from *N.meningitidis* (including serogroups A, B, C, W135 and Y), *S.pneumoniae* (including serotypes 1, 4, 5, 6B, 9V, 14,18C, 19F and 23F), *H.influenzae* type B, *Neisseria gonorrhoeae, Streptococcus agalactiae, Escherichia coli, Salmonella typhi, Streptococcus mutans, Cryptococcus neoformans, Moraxella calarrhalis, Klebsiella pneumoniae, Staphylococcus aureus,* and/or *Pseudomonas aeruginosa.*
Although the invention may be applied to any serogroup of *N.meningitidis*, it is preferred to use a capsular saccharide from serogroup A ("MenA"). The MenA capsular saccharide is particularly unstable in aqueous solution, meaning that special procedures need to be used to perform chemical manipulations (*e.g*. conjugation to carrier proteins) on this molecule. However, MenA saccharides modified according to the invention are found to be advantageously stable in aqueous solution.
The MenA capsular polysaccharide {→6)-D-Mar*p*NAc(3/4OAc)-α-(1→$OPO_3$→} is composed of N-acetylmannosamine residues linked together by α1-6 phosphodiester bonds having the repeat units shown below.

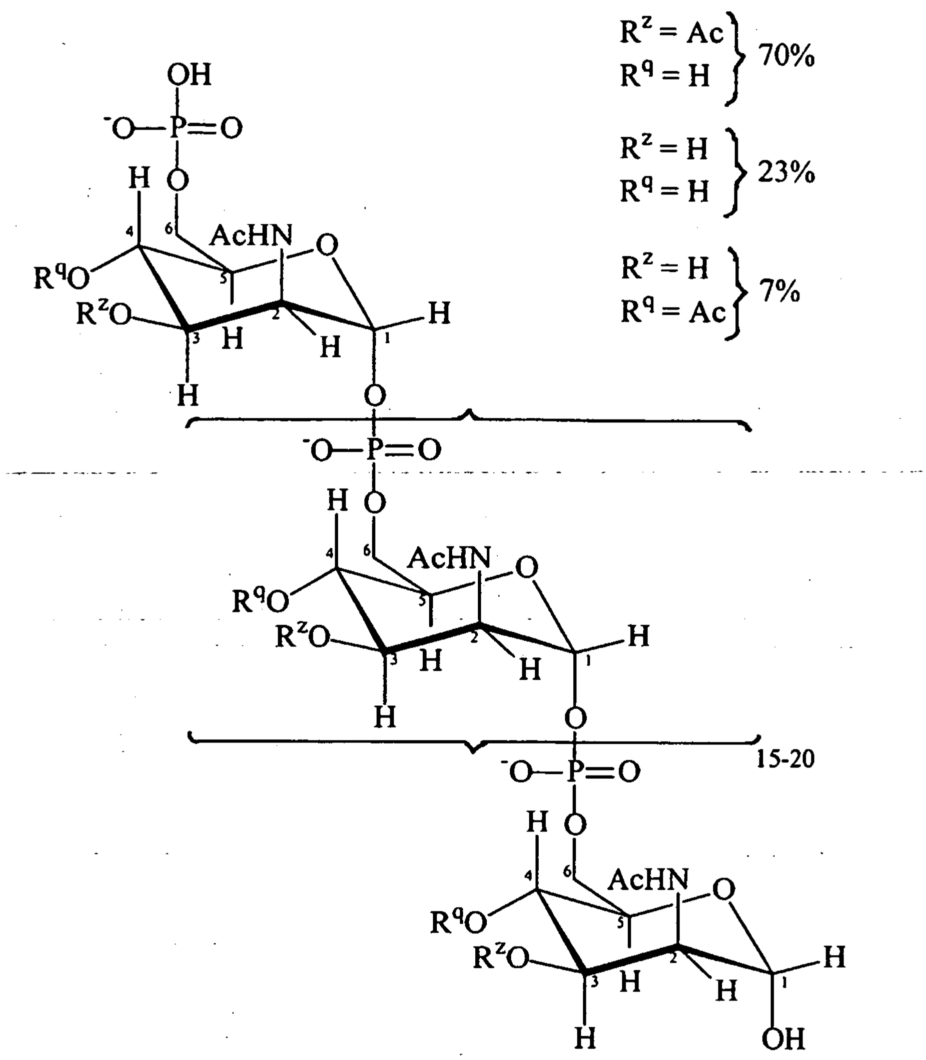

In accordance with the definitions above, 93% of the 4-positions are hydroxyl group positions, and 30% of the 3-positions are hydroxyl group positions. The terminal 1-hydroxy group also occupies a hydroxyl group position. The terminal 1-hydroxy group is a terminal anomeric hydroxyl group. The hydroxyl group which is part of the $-OP(O)(OH)O^-$ group is not a hydroxyl group position.

***Saccharide-protein conjugates***

The modified saccharides of the invention may be subjected to any usual downstream processing which is applied to saccharides (*e.g.* derivatisation, conjugation, fragmentation, *etc.*). To enhance immunogenicity, modified saccharides of the invention are preferably conjugated to a carrier protein. Conjugation to carrier proteins is particularly useful for paediatric vaccines [7] and is a well known technique [*e.g.* reviewed in refs. 8 to 16 *etc.*].

The invention thus provides a conjugate of a protein and a modified saccharide of the invention. The protein may be conjugated to the saccharide directly, or a linker may be used. Any suitable linker chemistry can be used. The improved stability of the modified polysaccharide advantageously allows a wide range of linkages to be used.

As described above, it is preferred that the modified capsular saccharide has at least one free hydroxyl group or amino group which can be used as a handle for subsequent linkage to a carrier protein.

A modified capsular saccharide having a free hydroxyl group may be obtained by selectively blocking hydroxyl groups on a capsular saccharide, or selectively de-blocking a modified capsular saccharide in which all the hydroxyl groups are blocked. Alternatively, a free hydroxyl group may be revealed by sizing a modified capsular saccharide. Preferably, the at least one free hydroxyl group is a terminal anomeric hydroxyl group. The terminal anomeric hydroxyl group is preferred as the free hydroxyl group because a terminal anomeric hydroxyl group may be revealed by sizing a modified capsular saccharide.

A modified capsular saccharide having a free amino group may be obtained by reductive amination of a terminal anomeric hydroxyl group, optionally followed by protection of the resulting -$NH_2$ group. The reductive amination reaction may be carried out before or after the modifying step of the present invention. Preferably, reductive amination is carried out before the modifying step of the present invention since the resulting -$NH_2$ group can be selectively protected/ deprotected in the presence of hydroxyl groups/blocking groups.

Direct linkages to the protein may comprise oxidation of the polysaccharide followed by reductive amination with the protein, as described in, for example, references 2 and 4.

Linkages via a linker group may be made using any known procedure, for example, the procedures described in references 3 and 5. A preferred type of linkage is a carbonyl linker, which may be formed by reaction of a free hydroxyl group of the modified saccharide with CDI [17, 18] followed by reaction with a protein to form a carbamate linkage. Another preferred type of linkage is an adipic acid linker, which may be formed by coupling a free -$NH_2$ group on the modified saccharide with adipic acid (using, for example, diimide activation), and then coupling a protein to the resulting saccharide-adipic acid intermediate. [12, 19, 20]. Other linkers include B-propionamido [21], nitrophenyl-ethylamine [22], haloacyl halides [23], glycosidic linkages [24], 6-aminocaproic acid [25], ADH [26], $C_4$ to $C_{12}$ moieties [27] *etc.*

Conjugation may involve: reduction of the anomeric terminus to a primary hydroxyl group, optional protection/ deprotection of the primary hydroxyl group; reaction of the primary hydroxyl group with CDI to form a CDI carbamate intermediate; and coupling the CDI carbamate intermediate with an amino group on a protein.

Scheme 2 shows two different examples of how a capsular saccharide may be conjugated to a carrier protein, in accordance with the present invention. In the first example, the protein is conjugated via a terminal hydroxyl group. In the second example, the protein is linked via a terminal amino group.

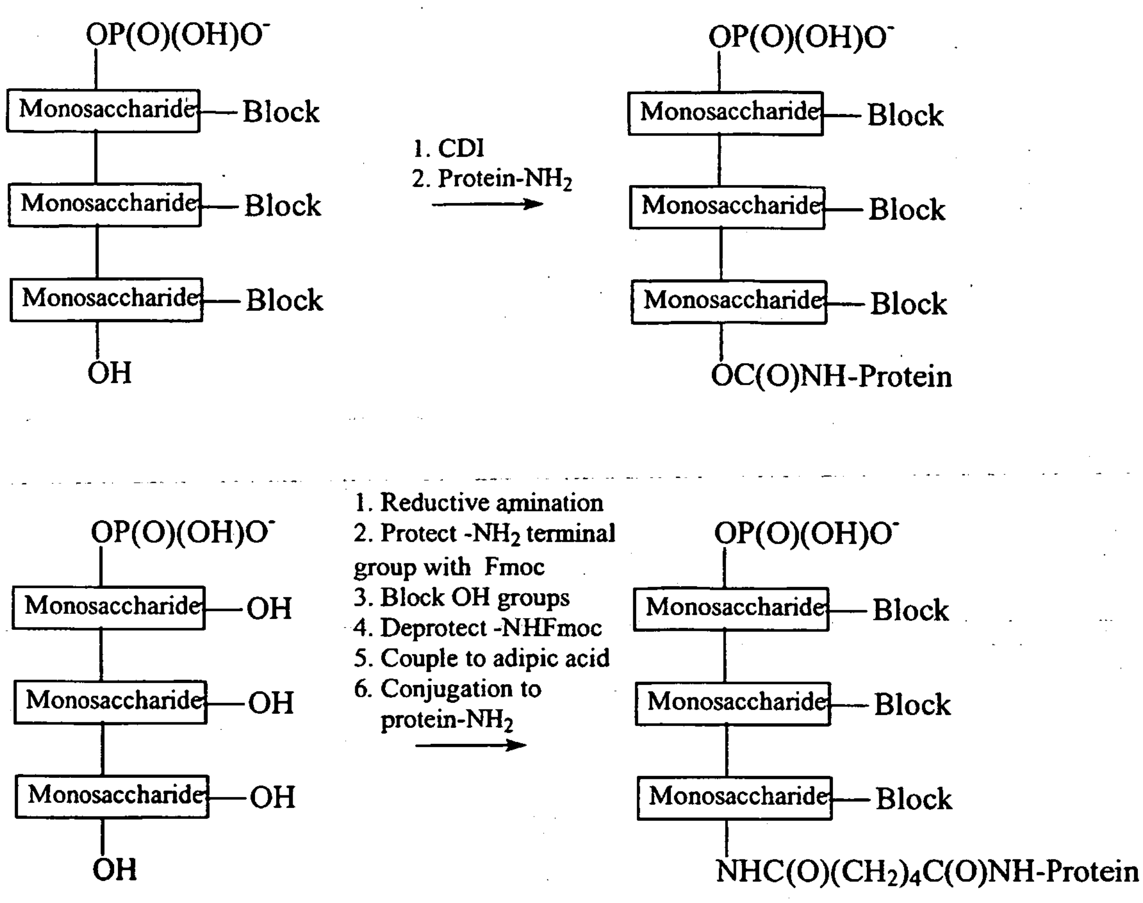


-Block is a blocking group

**Scheme 2**

Preferred carrier proteins are bacterial toxins or toxoids, such as diphtheria or tetanus toxoids. These are

commonly used in conjugate vaccines. The $CRM_{197}$ diphtheria toxoid is particularly preferred [28]. Other suitable carrier proteins include the *N.meningitidis* outer membrane protein [29], synthetic peptides [30,31], heat shock proteins [32,33], pertussis proteins [34,35], protein D from *H.influenzae* [36], cytokines [37], lymphokines [37], hormones [37], growth factors [37], toxin A or B from *C.difficile* [38], iron-uptake proteins [39] *etc*. It is possible to use mixtures of carrier proteins.

After conjugation, free and conjugated saccharides can be separated. There are many suitable methods, including hydrophobic chromatography, tangential ultrafiltration, diafiltration *etc*. [see also refs. 40, 41 *etc*.].

A single carrier protein may carry multiple different saccharides [42].

***Pharmaceutical compositions and methods***

The invention provides a pharmaceutical composition comprising (a) a modified saccharide of the invention and/or a conjugate of the invention, and (b) a pharmaceutically acceptable carrier.

Where a conjugate is present, the composition may also comprise free carrier protein [43].

'Pharmaceutically acceptable carriers' include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, trehalose [44] lipid aggregates (such as oil droplets or liposomes), and inactive virus particles. Such carriers are well known to those of ordinary skill in the art. The vaccines may also contain diluents, such as water, saline, glycerol, *etc*. Additionally, auxiliary substances, such as wetting or emulsifying agents, pH buffering substances, and the like, may be present. A thorough discussion of pharmaceutically acceptable excipients is available in Remington's Pharmaceutical Sciences.

Typically, the compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect. Direct delivery of the compositions will generally be parenteral (*e.g*. by injection, either subcutaneously, intraperitoneally, intravenously or intramuscularly, or delivered to the interstitial space of a tissue). The compositions can also be administered into a lesion. Other modes of administration include oral and pulmonary administration, rectal (suppositories), and transdermal or transcutaneous applications [*e.g*. ref. 45], needles, and hyposprays. Dosage treatment may be a single dose or a multiple dose schedule (e.g. including booster doses).

The composition of the invention is preferably sterile, buffered, and/or pyrogen-free.

The composition is preferably an immunogenic composition (*e.g*. a vaccine). Vaccines based on saccharides or saccharide-protein conjugates are well known in the art.

Immunogenic compositions comprise an immunologically effective amount of saccharide antigen, as well as any other of other specified components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e.g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e.g*. including booster doses). The vaccine may be administered in conjunction with other immunoregulatory agents.

The immunogenic composition may include an adjuvant. Preferred adjuvants to enhance effectiveness of the composition include, but are not limited to: (A) aluminium compounds e.g. aluminium hydroxides (*e.g*. oxyhydroxides), aluminium phosphates (*e.g*. hydroxyphosphates, orthophosphates), aluminium sulphates, *etc*. [*e.g.* see chapters 8 & 9 of ref. 46]), or mixtures of different aluminium compounds, with the compounds taking any suitable form (*e.g*. gel, crystalline, amorphous *etc*.), and with adsorption being preferred; (B) MF59 (5% Squalene, 0.5% Tween 80, and 0.5% Span 85, formulated into submicron particles using a microfluidizer) [see Chapter 10 of ref. 46; see also ref. 47]; (C) liposomes [see Chapters 13 and 14 of ref. 46]; (D) ISCOMs [see Chapter 23 of ref. 46], which may be devoid of additional detergent [48]; (E) SAF, containing 10% Squalane, 0.4% Tween 80, 5% pluronic-block polymer L121, and thr-MDP, either microfluidized into a submicron emulsion or vortexed to generate a larger particle size emulsion [see Chapter 12 of ref. 46]; (F) Ribi™ adjuvant system (RAS), (Ribi Immunochem) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (G) saponin adjuvants, such as QuilA or QS21 [see Chapter 22 of ref. 46], also known as Stimulon™; (H) chitosan [*e.g*. 49]; (I) complete Freund's adjuvant (CFA) and incomplete Freund's adjuvant (IFA); (J) cytokines, such as interleukins (*e.g*. IL-1, IL-2, IL-4, IL-5, IL-6, IL-7, IL-12, *etc*.), interferons (*e.g*. interferon-ã), macrophage colony stimulating factor, tumor necrosis factor, *etc.* [see Chapters 27 & 28 of ref. 46]; (K) microparticles (*i.e.* a particle of ~100nm to ~150ìm in diameter, more preferably ~200nm to ~30ì m in diameter, and most preferably ~50nm to ~10ìm in diameter) formed from materials that are biodegradable and non-toxic

(*e.g*. a poly(á-hydroxy acid), a polyhydroxybutyric acid, a polyorthoester, a polyanhydride, a polycaprolactone *etc*.); (L) monophosphoryl lipid A (MPL) or 3-O-deacylated MPL (3dMPL) [*e.g*. chapter 21 of ref. 46]; (M) combinations of 3dMPL with, for example, QS21 and/or oil-in-water emulsions [50]; (N) oligonucleotides comprising CpG motifs [51] *i.e*. containing at least one CG dinucleotide, with 5-methylcytosine optionally being used in place of cytosine, and/or CI motif; (O) a polyoxyethylene ether or a polyoxyethylene ester [52]; (P) a polyoxyethylene sorbitan ester surfactant in combination with an octoxynol [53] or a polyoxyethylene alkyl ether or ester surfactant in combination with at least one additional non-ionic surfactant such as an octoxynol [54]; (Q) an immunostimulatory oligonucleotide (*e.g*. a CpG oligonucleotide) and a saponin [55]; (R) an immunostimulant and a particle of metal salt [56]; (S) a saponin and an oil-in-water emulsion [57]; (T) a saponin (e.g. QS21) + 3dMPL + IL-12 (optionally + a sterol) [58]; (U) *E.coli* heat-labile enterotoxin ("LT"), or detoxified mutants thereof, such as the K63 or R72 mutants [*e.g*. Chapter 5 of ref. 59]; (V) cholera toxin ("CT"), or detoxified mutants thereof [*e.g*. Chapter 5 of ref. 59]; and (W) monophosphoryl lipid A mimics, such as aminoalkyl glucosaminide phosphate derivatives *e.g*. RC-529 [60]; (X) polyphosphazene (PCPP); (Y) a bioadhesive [61] such as esterified hyaluronic acid microspheres [62] or a mucoadhesive selected from the group consisting of cross-linked derivatives of poly(acrylic acid), polyvinyl alcohol, polyvinyl pyrollidone, polysaccharides and carboxymethylcellulose; or (Z) other substances that act as immunostimulating agents to enhance the effectiveness of the composition [*e.g*. see Chapter 7 of ref. 46]. Alum (especially aluminium phosphates and/or hydroxides) is a preferred adjuvant.

Muramyl peptides include N-acetyl-muramyl-L-threonyl-D-isoglutamine (thr-MDP), N-acetylnormuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE), *etc.*

Once formulated, the compositions of the invention can be administered directly to the subject. The subjects to be treated can be animals; in particular, human subjects can be treated. The vaccines are particularly useful for vaccinating children and teenagers.

Vaccines according to the invention may either be prophylactic (*i.e*. to prevent infection) or therapeutic (*i.e*. to treat disease after infection), but will typically be prophylactic.

As well as modified saccharides, the composition may comprise further antigenic components. For instance, the composition may include one or more further saccharides (whether or not modified according to the invention). For instance, the composition may comprise saccharides from serogroups C, W135 and Y of *N.meningitidis* (*e.g*. in addition to a modified MenA saccharide). These will typically be conjugated to carrier proteins, and saccharides from different serogroups of *N.meningitidis* may be conjugated to the same or different carrier proteins. Where a mixture comprises capsular saccharides from both serogroups A and C, it is preferred that the ratio (w/w) of MenA saccharide:MenC saccharide is greater than 1 *(e.g.* 2:1, 3:1, 4:1, 5:1, 10:1 or higher). Improved immunogenicity of the MenA component has been observed when it is present in excess (mass/dose) to the MenC component. [63]

The composition may also comprise protein antigens.

Antigens which can be included in the composition of the invention include:

- antigens from *Helicobacter pylori* such as CagA [64 to 67], VacA [68, 69], NAP [70, 71, 72], HopX *[e.g.* 73], HopY [*e.g*. 73] and/or urease.
- a protein antigen from *N.meningitidis* serogroup B, such as those in refs. 74 to 80, with protein '287' (see below) and derivatives (*e.g*. 'ÄG287') being particularly preferred.
- an outer-membrane vesicle (OMV) preparation from *N.meningitidis* serogroup B, such as those disclosed in refs. 81, 82, 83, 84 *etc.*
- a saccharide antigen from *N.meningitidis* serogroup C, such as the oligosaccharide disclosed in ref. 85 from serogroup C [see also ref. 86].
- a saccharide antigen from *Streptococcus pneumoniae* [*e.g.* 87, 88, 89].
- an antigen from hepatitis A virus, such as inactivated virus [*e.g*. 90, 91].
- an antigen from hepatitis B virus, such as the surface and/or core antigens [*e.g*. 91, 92].
- an antigen from hepatitis C virus *[e.g.* 93].
- an antigen from *Bordetella pertussis*, such as pertussis holotoxin (PT) and filamentous haemagglutinin (FHA) from *B.pertussis*, optionally also in combination with pertactin and/or agglutinogens 2 and 3 [*e.g.* refs. 94 & 95].
- a diphtheria antigen, such as a diphtheria toxoid [*e.g.* chapter 3 of ref. 96] *e.g.* the $CRM_{197}$ mutant [*e.g*. 97].
- a tetanus antigen, such as a tetanus toxoid [*e.g.* chapter 4 of ref. 96].
- a saccharide antigen from *Haemophilus influenzae B* [*e.g.* 86].
- an antigen from *N.gonorrhoeae* [*e.g.* 74, 75, 76].
- an antigen from *Chlamydia pneumoniae* [*e.g.* 98, 99, 100, 101, 102, 103, 104].
- an antigen from *Chlamydia trachomatis* [*e.g*. 105].
- an antigen from *Porphyromonas gingivalis* [*e.g.* 106].
- polio antigen(s) [*e.g*. 107, 108] such as IPV or OPV.
- rabies antigen(s) [*e.g*. 109] such as lyophilised inactivated virus [*e.g*. 110, RabAvert™].

- measles, mumps and/or rubella antigens [*e.g*. chapters 9, 10 & 11 of ref. 96].
- influenza antigen(s) [*e.g.* chapter 19 of ref. 96], such as the haemagglutinin and/or neuraminidase surface proteins.
- an antigen from *Moraxella catarrhalis* [*e.g.* 111].
- an antigen from *Streptococcus agalactiae* (group B streptococcus) *[e.g.* 112, 113].
- a saccharide antigen from *Streptococcus agalactiae* (group B streptococcus).
- an antigen from *Streptococcus pyogenes* (group A streptococcus) [e.g. 113, 114, 115].
- an antigen from *Staphylococcus aureus* [*e.g.* 116].
- an antigen from *Bacillus anthracis* [*e.g*. 117, 118, 119].
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen e.g. from parvovirus B19.
- a prion protein (*e.g*. the CJD prion protein)
- an amyloid protein, such as a beta peptide [120]
- a cancer antigen, such as those listed in Table 1 of ref. 121 or in tables 3 & 4 of ref. 122.

The composition may comprise one or more of these further antigens.

Toxic protein antigens may be detoxified where necessary (*e.g*. detoxification of pertussis toxin by chemical and/or genetic means [95]).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens.

Antigens are preferably adsorbed to an aluminium salt.

Antigens in the composition will typically be present at a concentration of at least 1 μg/ml each. In general, the concentration of any given antigen will be sufficient to elicit an immune response against that antigen.

As an alternative to using proteins antigens in the composition of the invention, nucleic acid encoding the antigen may be used [*e.g*. refs. 123 to 131]. Protein components of the compositions of the invention may thus be replaced by nucleic acid (preferably DNA *e.g.* in the form of a plasmid) that encodes the protein.

The invention also provides a method for raising an antibody response in a mammal, comprising administering a pharmaceutical composition of the invention to the mammal. The mammal is preferably a human. The human may be an adult or, preferably, a child. The antibody response is preferably protective against infection by *N.meningitidis* serogroup A.

The invention also provides a method for immunising a mammal, comprising administering a pharmaceutical composition of the invention to the mammal.

This invention also provides a modified saccharide of the invention, or a conjugate of the invention, for use as a medicament.

The invention also provides the use of a modified saccharide of the invention, or of a conjugate of the invention, in the manufacture of a medicament for preventing or treating a disease caused by capsulate bacteria. Diseases caused by *Neisseria* include meningitis, septicaemia and gonorrhoea. Diseases caused by *H.influenzae* include otitis media, bronchitis, pneumonia, cellulitis, pericarditis, and meningitis. Diseases caused by pneumococcus include meningitis, sepsis and pneumonia. The prevention and/or treatment of bacterial meningitis is thus preferred.

***Definitions***

The term "comprising" means "including" as well as "consisting" *e.g*. a composition "comprising" X may consist exclusively of X or may include something additional *e.g.* X + Y.

The term "about" in relation to a numerical value x means, for example, $x \pm 10\%$.

It will be appreciated that sugar rings can exist in open and closed form and that, whilst closed forms are shown in structural formulae herein, open forms are also encompassed by the invention.

**BRIEF DESCRIPTION OF DRAWINGS**

Figure 1 shows the *avDP* of Men A samples after incubation at 37, 49 and 57 ˚C plotted as a function of time (h).

Figure 2 shows the *avDP* of MenA-CDI-DMA samples after incubation at 37, 49, 57 ˚C plotted as a function of time (h)

Figure 3 shows a stacked plot of $^{31}P$ NMR 242.9 MHz spectra of MenA-CDI-DMA samples incubated at 57 ˚C for 0, 24, 48, 72, 96 hours. Some signal labels are indicated.

Figure 4 shows the *avDP* vs. time diagram to compare colorimetric and $^{31}P$ NMR analytical methods.

Figure 5 shows the *avDP* of native and modified MenA saccharides at 2-8˚C over time

Figure 6 shows labelled $^{1}H$ NMR 600 MHz spectrum of MenA-CDI-DMA at 298 K. Some assignments are indicated.

Figure 7 shows results of a competitive ELISA test performed using MenA, MenA-CDI and MenA-CDI-DMA oligosaccharides as coating agent. The concentration of competitors ranged from 10˚ to $10^{-7}$ mg/ml.

Figure 8 illustrates the reaction scheme for conjugation of MenA oligosaccharides.

Figure 9 shows the 600 MHz $^{1}H$ NMR spectrum at 25˚C of activated modified MenA. Some signal labels are indicated.

Figures 10A & 10B show hetero-correlate $^{1}H$,$^{13}C$ NMR spectra at 25˚C of activated modified MenA. In both Figure 10A & 10B, the X axis runs approximately from 5.7ppm at the left to 1.8ppm at the right. In Figure 10A, the Y axis runs approximately from 145ppm at the top to 185ppm at the bottom; in Figure 10B it runs approximately from 5ppm at the top to 105ppm at the bottom.

Figure 11 shows superimposed $^{1}H$ NMR spectra of activated modified MenA DP4 and activated native MenA DP4 (without the chemical modification by CDI and DMA).

Figure 12 shows a 243 MHz $^{31}P$ NMR spectrum at 25˚C of activated modified MenA.

Figures 13 and 14 show the appearance of free saccharide due to hydrolysis of conjugates stored at 37˚C over a four week period. Modified saccharides are shown as squares, natural as empty triangles.

Figure 15 shows the appearance of free saccharide due to hydrolysis of conjugates stored at 37˚C for four weeks at various pH. The left-hand column of each pair shows native oligosaccharide.

Figure 16 shows the anti-MenA-pS IgG titres induced by (left to right) lot 3, lot 5 and lot 002011 conjugates. Bars show 95% confidence limits.

Figure 17 shows IgG subclasses analysis of pooled sera from immunization with MenA modified and unmodified conjugates (lots 3, 5 & 002011). Values are $OD_{405nm}$ multiplied by 1000.

Figure 18 shows the results of a competitive ELISA using MenA pS as competitor. The Y axis shows $OD_{405nm}$ values multiplied by 1000. The X axis shows the reciprocal serum dilution. Unmodified MenA oligosaccharide is shown in circles; modified saccharides are shown in squares (lot 3) and in triangles (lot 5). Empty symbols show data without competitor polysaccharide; filled symbols show data in the presence of competitor polysaccharide.

## MODES FOR CARRYING OUT THE INVENTION

### *Modification of MenA oligosaccharide*

Capsular polysaccharide was purified from MenA and was hydrolysed to give MenA oligosaccharide. The polysaccharide (2 g) was hydrolyzed at 50˚C in 50 mM sodium acetate buffer, pH 4.75, at a polysaccharide concentration of 10 mg/mL for about 4 hours [86]. After hydrolysis, the solution was dried by rotary evaporation.

The oligosaccharide was activated using the reaction scheme shown above in Scheme 1. The oligosaccharide was dissolved in DMSO to give a saccharide concentration of 10 mg/mL. According to a molar ratio of oligosaccharide: CDI being 1:20, 21.262 g of CDI (Sigma™) was then added and the reaction mixture stirred for 16 hours at room temperature. The resulting MenA-CDI compound was purified by selective precipitation in a 80:20 (v/v) acetone:DMSO mixture followed by centrifugation. The efficiency of the activation reaction was calculated to be about 67.9% by determining the ratio of free imidazole to bonded imidazole.

In the second reaction step, the MenA-CDI oligosaccharide was solubilised in DMSO at a saccharide concentration of about 10 mg/mL. According to a molar ratio of MenA-CDI unit:DMA being 1:100, 36.288 g of 99% dimethylamine

hydrochloride (Sigma™) was added and the reaction mixture stirred for 16 hours at room temperature. The reaction product was freeze-dried and re-solubilised in 10 mg/mL water solution.

To remove the low molecular weight reaction reagent (in particular the dimethylamine (DMA)) from the oligosaccharide preparation, a dialysis step was performed through a 3.5 kDa MWCO membrane (Spectra/Por®). Four dialysis steps were carried out: (i) 16 hours vs. 2 L of 1 M sodium chloride (dialysis factor 1:20), (ii) 16 hours vs. 2 L of 0.5 M sodium chloride (dialysis factor 1:20), (iii) and (iv) 16 hours vs. 2 L of WFI (dialysis factor 1:20). To improve the purification a diafiltration step was also performed through a 1 kDa MWCO membrane (Centricon™).

The purified MenA-CDI-DMA product was buffered at pH 6.5 in 25 mM L-histidine (Fluka™).

Stability of the MenA and MenA-CDI-DMA products was assessed by using colorimetric and NMR methods to determine their average degree of polymerisation (*avDP*). Samples were incubated in glass vials in 25 mM His buffer, pH 6.5, for 96 hours at one of three temperatures (37, 49, or 57˚C) and, at the end of the incubation period, the samples were stored at 4˚C.

***Colorimetric stability study***

The chemical *avDP* is expressed by the ratio $[P_t]/[P_{me}]$, where $[P_t]$ is the total phosphorus concentration and $[P_{me}]$ is the terminal monoester phosphate concentration. $[P_t]$ was determined colorimetrically as described in ref. 132. $[P_{me}]$ was determined by measuring the inorganic phosphate $P_i$ released by enzymatic reaction with potato acid phosphatase [133].

Figures 1 and 2 show the *avDPs* of MenA and MenA-CDI-DMA as a function of time (*t*).

Kinetic constants (k) of saccharide hydrolysis (seen in Figures 1 and 2 as the drop in avDP) were analysed as described in reference 134. Two distinct aspects of *k* were analysed:

- *k* as a function of *avDP* and *t* in the standard kinetic equation; and then
- *k* as function of frequency factor (A), activation energy ($\Delta G_a$) and temperature (*T*) in the Arrhenius equation.

It was assumed that hydrolysis proceeded to completion with satisfactory first-order kinetics:

$$\frac{d\,avDP}{dt} = -k\,avDP \qquad avDP = avDP_0 \exp(-kt)$$

where $avDP_0$ = *avDP* at *t* = 0.

The logarithmic form is:

$$\ln avDP = \ln avDP_0 - kt$$

*k* is defined by the slope of ln *avDP* = f(*t*) plot.

The Arrhenius equation indicates the correlation between the kinetic constants at various temperature values and the activation energy for the hydrolysis reaction:

$$k = A \exp(-\Delta G_a / RT)$$

$$\ln k = \ln A - \Delta G_a / RT$$

where R = $8.314 \times 10^{-3}$ KJ/mol K.

$\Delta G_a$ is calculated from the slope of straight line obtained by plotting ln *k* as a function of reciprocal temperature (1/T). In this study we analysed only the total activation energy of hydrolysis reaction, without the separation of the single contributions from the activation enthalpy and activation entropy ($\Delta G_a = \Delta H_a + T\Delta S_a$).

Table 1 summarises the colorimetric *avDP* data and the kinetic constants of the hydrolysis reaction at various temperatures:

| | T (K) | *avDP* | | | | | k ($s^{-1}$) |
|---|---|---|---|---|---|---|---|
| | | 0 h | 24 h | 48 h | 72 h | 96 h | |
| **MenA** | 310 | 21.453 | 17.452 | 14.197 | 11.550 | 9.396 | 2.4 x $10^{-6}$ |
| | 322 | 21.453 | 14.028 | 9.173 | 5.998 | 3.922 | 4.9 x $10^{-6}$ |
| | 330 | 21.453 | 10.956 | 5.595 | 2.857 | 1.459 | 7.8 x $10^{-6}$ |
| **MenA- CDI-DMA** | 310 | 21.453 | 21.192 | 20.994 | 19.524 | 18.640 | 1.9 x $10^{-7}$ |
| | 322 | 21.453 | 22.410 | 19.127 | 17.472 | 15.491 | 9.2 x $10^{-7}$ |
| | 330 | 21.453 | 20.227 | 16.555 | 13.864 | 11.600 | 1.8 x $10^{-6}$ |

Arrhenius plots of rate constants obtained at 37, 49, and 57°C, indicate that the activation energy of hydrolysis reaction in 25 mM His buffer, pH 6.5, is 50.1 KJ/mol (12.0 Kcal/mol) for MenA and 94.9 KJ/mol (22.7 Kcal/mol) for MenA-CDI-DMA. Standard errors, estimated by linear least squares regression of Arrhenius plots, are ± 5.0 KJ/mol (± 1.2 Kcal/mol) for $\Delta G_a$ values. Thus, the modified polysaccharide of the invention is nearly twice as stable as its unmodified counterpart.

***NMR stability study***

In order to verify the *avDP* obtained by the colorimetric method, $^{31}$P NMR analytical experiments were carried out. The *avDP* data were calculated by the integration ratio between $P_{me}$ and $P_{in\ chain}$ signals (see Figure 3).

$^{1}$H and $^{31}$P NMR samples were prepared by dissolving lyophilized oligosaccharides in 0.75 ml of 99.9% $D_2O$ (Aldrich™) to give 10-15 mM concentrated solutions. In all experiments, 5 mm Wilmad™ NMR tubes were used. NMR spectra were recorded at 298 K on a Bruker™ NMR Spectrometer Avance DRX 600 MHz with a BGU unit. A 5 nun TBI triple resonance probe with self shielded z-gradients was used. For processing data the Bruker XWINNMR 3.0 software was used. $^{1}$H standard spectral acquisition conditions are to collect 32 k data points over a spectral window of 6000 Hz with 4 scans. $^{1}$H NMR spectra were Fourier-transformed after applying a 0.1 Hz line broadening function and referenced relative to the acetate anion resonance at 1.91 ppm or that of monodeuterated water at 4.72 ppm. $^{31}$P standard spectral acquisition conditions are to collect 32 k data points over a spectral window of 3000 Hz with 128 scans. 2.0 Hz line broadening function was used.

As shown in Figure 4, the colorimetric and $^{31}$P NMR methods agree for all temperature values, with only a slight down-translation being evident (no effects in ln *avDP* = f(*t*)).

The results of the colorometric and $^{31}$P NMR methods of analysis are summarised in Table 2:

| Analytical method | T (K) | *avDP* | | | | | k ($s^{-1}$) |
|---|---|---|---|---|---|---|---|
| | | 0 h | 24h | 48h | 72h | 96h | |
| **Colorimetric determination** | 310 | 21.453 | 21.192 | 20.994 | 19.524 | 18.640 | 1.9 x $10^{-7}$ |
| | 322 | 21.453 | 22.410 | 19.127 | 17.472 | 15.491 | 9.2 x $10^{-7}$ |
| | 330 | 21.453 | 20.227 | 16.555 | 13.864 | 11.600 | 1.8 x $10^{-6}$ |
| **$^{31}$P-NMR determination** | 310 | 20.407 | 19.573 | 19.170 | 18.450 | 18.253 | 3.3 x $10^{-7}$ |
| | 322 | 20.407 | 16.986 | 14.836 | 12.556 | 10.051 | 2.0 x $10^{-6}$ |
| | 330 | 20.407 | 15.984 | 12.123 | 9.860 | 8.079 | 2.7 x $10^{-6}$ |

By improving the analysis by the spectral shape of $P_{me}$, two different molecular species are seen to arise (see Figure 3). A lower kinetic constant rate is evident for the up-field signal.

By extrapolation of acquired data at lower temperature (e.g. the typical storage temperature of 2 to 8°C), it is possible to extrapolate these data to a 2 year time scale (see Figure 5).

Comparing the results obtained by these investigations on the stability of MenA and MenA-CDI-DMA, a significant increase of stability of the modified product is observed. The extrapolation to a longer time scale indicates that the degradation of the MenA-CDI-DMA is sufficiently reduced to allow distribution of the product for 2 years.

***Structural characterization***

Figure 6 shows a $^1H$ NMR spectrum of a MenA-CDI-DMA sample at 298 K with indicative signal assignments. The NMR profile suggests high similarity between the MenA oligosaccharide and the MenA-CDI-DMA oligosaccharide. $^1H$-methyl DMA signals are seen in the 2.6-3 ppm spectral region. The 2.73 ppm peak corresponds to the $^1H$-methyl of free DMA, as a residual reagent in solution. The 2.91 ppm peak was tentatively assigned as the $^1H$-methyl DMA bond to oligosaccharide chain.

The $^{31}P$ NMR profiles are similar to the MenA oligosaccharide. Only a short down-field shift of the $P_{me}$ signal is observed (see Figure 3).

The modified saccharides of the invention are therefore structurally similar to their native counterparts, which should mean that antigenicity and immunogenicity are unaffected.

***Competitive ELISA assays***

A competitive ELISA assay was used to correlate MenA, MenA-CDI and MenA-CDI-DMA oligosaccharides with their ability to displace specific antibodies. The % of inhibition plotted as a function of competitor concentration (mg/mL) are shown in the Figure 7. All samples showed a similar behaviour, reaching about 100% of inhibition at $10^{-1}$ mg/mL competitor concentration.

This confirms that modification of saccharides using the invention does not result in loss of antigenicity.

***Conjugation***

The modified MenA saccharide (MenA-CDI-DMA) was conjugated to $CRM_{197}$ protein by the process summarised in Figure 8. The basic steps in the conjugation process are:

- hydrolysis of MenA polysaccharide to give oligosaccharide fragments
- sizing of the oligosaccharide fragments
- reductive amination of terminal aldehyde groups on the sized oligosaccharides
- protection of terminal $-NH_2$ groups by Fmoc group before the CDI reaction
- intrinsic de-protection of $-NH_2$ groups during the DMA reaction
- activation of terminal $-NH_2$ groups by SIDEA (N-hydroxysuccinimide adipic acid)
- covalent attachment to $CRM_{197}$ protein

a) Hydrolysis

MenA polysaccharide was hydrolysed in 50 mM sodium acetate buffer, pH 4.75 for about 3 hours at 73°C. Hydrolysis was controlled in order to obtain oligosaccharides with an average degree of polymerisation (DP) of approximately 15, as determined by the (w/w) ratio between the total organic phosphorous and the monoester phosphate.

b) Sizing

This step selects a defined population of oligosaccharides generated during the hydrolysis process. The hydrolysate obtained above was ultrafiltered through a 30kDa cut-off membrane (12 diafiltration volumes of 5 mM acetate buffer, pH 6.5) to remove the long-length chains in the retentate moiety.

c) Introduction of a primary amino group at the reducing terminus

Ammonium acetate was added to the sized oligosaccharide solution for a final concentration of 300 g/L, and sodium cyano-borohydride was added to a final concentration of 73 g/L. After adjusting the pH to $6.5 \pm 0.2$, the mixture was incubated at 37°C for 5 days.

The amino-oligosaccharides were then purified by ultrafiltration through a 3kDa cut-off membrane using 13 volumes of 0.5 M NaCl. This step removes short-length saccharide chains (DP<6-7) giving a final degree of average polymerisation of ~15.

The retentate moiety was diafiltered with 4 volumes of 10mM TAB (tetrabutyl ammonium bromide) and then with 7 volumes of $H_2O$ to exchange $Na^+$ to $TAB^+$. The positive organic ion improves the saccharide's solubility in DMSO (required for the next derivatisation steps) to about 10 g/L.

Purified' oligosaccharides were dried with a rotary evaporator to remove water and then were solubilized in DMSO solvent at concentration of about 10g/L.

The purified amino-oligosaccharide solution was analysed for phosphorous content by the procedure of Chen [132] and for the amount of introduced amino groups by the procedure of Habbeb [135].

As an alternative to ultrafiltration to remove short-chain saccharides, a Q-Sepharose Fast Flow column was used, but a further exchange process onto a SP-Sepharose (Pharmacia™) column is then needed to effect the $Na^+$/$TAB^+$ conversion.

d) Protection of terminal amino group with Fmoc reagent

The amino-oligosaccharides were reacted with Fmoc-OSu (N-9-Fluorenylmethoxycarbonyloxy) (Sigma) according to the molar ratio -$NH_2$:Fmoc-OSu = 1:20. The mixture was incubated overnight under stirring at room temperature and was precipitated with acetone (80% v/v final concentration). The precipitate was collected by centrifugation and washed several times with acetone to remove unreacted Fmoc-OSu reagent.

e) Stabilizing reaction with CDI and DMA reagents

The protected amino-oligosaccharides were solubilised in DMSO at 10g/L and added to CDI at a molar ratio of CDI:total phosphorous = 20:1. The mixture was incubated overnight under stirring at room temperature and was precipitated with acetone (80% v/v final concentration). The precipitate was collected by centrifugation and washed several times with acetone to remove unreacted CDI reagent.

The product obtained above was solubilised in DMSO at 10g/L and added to a solution of DMA in ethanol (about 5.6 M) according to the molar ratio of DMA:total phosphorous = 20:1. The mixture was incubated overnight under stirring at room temperature and was precipitated with acetone (80% v/v final concentration). The precipitate was collected by centrifugation and washed several times with acetone to remove unreacted DMA.

The purified oligosaccharides were then dried under vacuum to remove traces of organic solvents.

f) Chromatographic ionic exchange

The dried oligosaccharide was solubilised in water at 10 g/L and loaded onto a SP-Sepharose Fast Flow (Pharmacia™) column equilibrated in 1 M NaCl, in order to perform the $Tab^+$/$Na^+$ exchange. The column was then washed with 5 column volumes (CV) of water to recuperate traces of product adsorbed to resin. The oligosaccharide was then dried with rotary evaporator to remove water.

g) Derivatisation to active ester

The dried product was solubilised in water at a 40 mM amino group concentration, then 9 volumes of DMSO were added followed by TEA (triethyl-amine) at a final concentration of 200 mM. To the resulting solution, adipic acid N-hydroxysuccinimido diester (SIDEA) was added for a final concentration of 480 mM.

The reaction was maintained under stirring at room temperature for 2 hours, then the activated oligosaccharide was precipitated with acetone (80% v/v final concentration). The precipitate was collected by centrifugation and washed several times with acetone to remove unreacted SIDEA.

The purified oligosaccharides were then dried under vacuum to remove the solvent.

The amount of active ester groups introduced into the oligosaccharide structure was determined by a colorimetric method as described in reference 136.

The activated oligosaccharide was analysed by $^1H$ NMR as described above to confirm the chemical modifications. The proton NMR profiles established in previous experiments were used to evaluate several lots of product. The saccharide signals were assigned by inspection of 1D (Figure 9) and 2D hetero-correlate ($^1H$, $^{13}C$; Figures 10A & 10B) NMR spectra and were shown to be characteristic of the modified MenA.

About 5 mg of each sample were dissolved in 750$\mu$L $D_2O$ and the spectra were recorded on a Bruker Avance 600 MHz spectrometer.

Inspection of the ratio of $CH_3^{DMA}$ groups and $H_1^{saccharide\ ring}$ provided a stabilizing reaction yield of between 70% and 75 %.

The modified saccharide proton profile is maintained, and substantial modifications concerning the O-acetyl status and the structural conformation are not evident from the NMR analysis. However, the carbamate groups change the local magnetic field and thus the assignment of $^1H$ NMR spectrum is complicated.

In Figure 11, superimposed spectra of modified and native MenA oligosaccharides are shown. A down-field shift of $H_3$, $H_4$ and $H_2$ signals is evident because the carbamate groups in $C_3$ and $C_4$ ring position are nearer than other nuclei, as for instance $H_1$. Other shifts are suggested from the spectra but their assignment is not completely certain.

Figure 12 shows that chemical derivatisation does not cause any change in the $^{31}P$ NMR spectrum.

h) Conjugation to $CRM_{197}$

The dried activated oligosaccharide was added to a 45 mg/mL solution of $CRM_{197}$ in 10 mM phosphate buffer, pH 7.2, according to a molar ratio of ester groups:protein = 12:1. The reaction was maintained under stirring at room temperature overnight and the obtained conjugate was purified by tangential ultrafiltration through 30kDa cut-off membrane using 50 volumes of phosphate buffer 10 mM, pH 7.2. The product was sterile filtered and stored at -20˚C until vaccine formulation.

The purified conjugate was analysed for protein content (microBCA Protein Assay), saccharide content (colorimetric determination of phosphorous), free saccharide content (chromatographic analysis), HPLC profile (on TSKgel G4000SWXL 7.5 mm IDx30cm), NMR profile and SDS-PAGE.

***Time-dependent stability of the conjugate***

The stability of the $CRM_{197}$ conjugate of the MenA-CDI-DMA oligosaccharide was assessed by monitoring the appearance of free saccharide in solution, due to hydrolysis, during four weeks of storage at 37˚C, in comparison to a CRM197 conjugate of unmodified MenA oligosaccharide.

Free (*i.e.* non-conjugated) saccharide was determined uding reversed-phase chromatography on ISOLUTE™ C4 cartridge column (IST™) to isolate the non-conjugated chains, and then by permeated saccharide with HPAE-PAD chromatography.

Total saccharide (*i.e.* both conjugated and non-conjugated) was determined by a method for the quantitative analysis of N-acetyl mannosammine phosphate, which uses high-performance anion-exchange chromatography with pulsed-amperometric detection (HPAE-PAD) [137].

The ratio of unconjugated saccharide to total saccharide was expressed as a percentage (%FS).

In a first experiment, %FS developed as follows (Figure 13):

| Time (days) | 0 | 7 | 14 | 21 | 28 |
|---|---|---|---|---|---|
| Modified | 16.8 | 14.7 | 23.8 | 21.7 | 23.3 |
| Natural | 11.0 | - | 27.0 | 36.6 | 39.5 |

In a second experiment, results were as follows (Figure 14):

| Time (days) | 0 | 7 | 14 | 21 | 28 |
|---|---|---|---|---|---|
| Modified | 1.8 | 1.5 | 4.2 | 4.9 | 8.2 |
| Natural | 4.8 | 5.5 | 19.3 | 22.7 | 28.3 |

The modified MenA oligosaccharide conjugate is clearly much more resistant to hydrolysis than its natural counterpart at elevated temperatures. After 28 days at 37˚C, for instance, the percentage of released saccharide is 6.4 % for the modified oligosaccharide vs. 23.5 % for the natural sugar.

In further work to test lot-to-lot consistency using the modified MenA saccharide, the appearance of free saccharide from conjugates was monitored for 8 weeks at 37˚C. Results for three lots were:

| Time (days) | 0 | 7 | 14 | 28 | 56 |
|---|---|---|---|---|---|
| Lot A | 1.7 | 2.9 | 3.2 | 5.8 | 8.7 |
| Lot B | 1.0 | 4.2 | 4.5 | 6.5 | 10.9 |
| Lot C | 2.2 | 4.5 | 5.4 | 8.2 | 11.4 |

The modified conjugate is thus stable over an extended period. A free saccharide level of less than 12% is well within acceptable limits, even at above-normal temperature.

***pH-dependent stability of the conjugate***

Stability of the conjugates of modified and unmodified MenA oligosaccharides was tested by monitoring the

appcarance of free saccharide at different pH between 6.0 and 8.0 after being stored at 37˚C for 28 days. Modified (Lot 5) and unmodified (Lot RS040101) oligosaccharides were compared and the increases in free saccharide (Ä%FS) between days 0 and 28 were as follows (Figure 15):

| pH * | 6.0 | 6.5 | 7.0 | 7.5 | 8.0 |
|---|---|---|---|---|---|
| Modified | 10.3 | 5.4 | 8.9 | 8.3 | 26.1 |
| Native | 43.5 | 30.1 | 36.1 | 20.3 | 30.5 |
| * pH±0.1 | | | | | |

The modified MenA conjugate thus shows a much lower hydrolysis reaction rate than conjugate of the native MenA oligosaccharide in the pH range 6.5-7.5. At pH 8.0, where carbamate stabilising groups are removed, the effect is less marked.

***Immunogenicity of modified conjugates***

Purified $CRM_{197}$ conjugates of the modified MenA oligosaccharide were used to immunise mice in order to verify that the modification does not remove the saccharide's immunogenicity.

The vaccine was formulated to give a single human dose (SHD) of 10μg saccharide in a 0.5ml volume. Two formulations were made: a liquid formulation and a lyophilized formulation. Both contain an aluminium phosphate adjuvant at 0.6mg $Al^{3+}$/ml in the final dosage form, with an aqueous suspension of the adjuvant being used to reconstitute the lyophilised formulation.

The liquid formulation of the modified oligosaccharide conjugate was compared to the lyophilised formulation of the native (*i.e.* unmodified) oligosaccharide conjugate.

Mice were immunised with 1/5 of the SHD, the vaccines being diluted with saline before each immunization. 10 Balb/c mice (female, 6-8 weeks old) per immunisation group were injected subcutaneously with 0.5 ml of the vaccine at time zero and then four weeks later. Bleedings were performed before the first immunisation and at week 6 (pre and post-II sera), with sera being stored at -70˚C.

Anti-polysaccharide titres

Specific anti-MenA polysaccharide total IgG antibodies were determined in the sera of immunized animals according to the CDC procedure for MenA human sera analysis [138], adapted for animal sera analysis, with some minor changes.

Each individual mouse serum was analyzed in duplicate by a titration curve. GMT was calculated for immunization groups. Anti-MenA polysaccharide titre was expressed in Mouse Elisa Units (MEU), with software based on the Reference Line Assay Method being used for MEU calculation.

IgG subclasses analysis was performed with pooled post-II sera of the immunization groups, using alkaline phosphatase-anti mouse IgG1, or IgG2a, or IgG2b or IgG3 (Zymed) as secondary conjugate in the ELISA procedure. Titres were expressed as $OD_{405nm}$ obtained at a dilution 1:3200 of the pooled post-II sera after 30 minutes of substrate development.

Figure 16 shows anti-MenA-pS IgG titres (GMT) induced by the two lots of MenA modified conjugate (lots 3 and 5) compared to the lyophilised unmodified MenA conjugate (lot 002011) using aluminium phosphate adjuvant. Both modified conjugates induced a titre very similar to that induced by the unmodified MenA conjugate.

Figure 17 shows IgG subclasses analysis of the pooled sera (diluted 1:3200) from immunisation with modified and unmodified MenA conjugates. The most represented subclass in all sera is IgG1, the subclass predominantly induced in mice by T-dependent antigens when presented as proteins. Because the MenA capsular saccharide is naturally a T-independent antigen which is not able to induce immunological memory, this shows that the conjugation achieves its purpose.

Anti-MenA pS titre specificity was determined by a competitive ELISA using MenA pS as competitor at a final concentration of 25μg/ml. As shown in Figure 18, there is very good inhibition of the titre induced by the modified and unmodified conjugates, indicating that all conjugates were able to induce anti-MenA pS-specific titres.

Serum bactericidal assay (SBA) against *N.meningitidis* serogroup A

The functionality of antibodies induced by immunisation with the conjugates was analyzed in an *in vitro* bactericidal assay to measure complement-mediated lysis of bacteria.

Pooled post-II sera for each immunisation group were used. They were inactivated for 30 minutes at 56˚C before the use in the assay. 25% baby rabbit complement was used as the source of complement (Pel Freeze). The bactericidal titre was expressed as the reciprocal serum dilution yielding 50% killing of the bacteria. Activity against two serogroup A strains was tested: F8238 & F6124.

Titres were as follows:

| Target strain | Lot 3 | Lot 5 | Lot 002011 |
|---|---|---|---|
| F8238 | 2048-4096 | 2048 | 4096-8192 |
| F6124 | 4096 | 2048 | 4096 |

Therefore all three conjugates induce good bactericidal titres against both strains, and the titres induced by the modified oligosaccharides are not significantly lower than those obtained using the native sugar structures. Advantageously, however, the modified oligosaccharides are significantly more stable than the native oligosaccharides. The invention therefore provides antigens which retain the immunogenic potential of the native MenA capsular saccharide, but which offer improved resistance to hydrolysis during storage.

It will be understood that the invention has been described by way of example only and modifications may be made whilst remaining within the scope of the appended claims.

**REFERENCES** (the contents of which are hereby incorporated by reference)

[1] US patent 4,711,779
[2] US patent 4,761,283
[3] US patent 4,882,317
[4] US patent 4,356,170
[5] US patent 4,695,624
[6] Nilsson & Svensson (1979) Carbohydrate Research 69: 292-296)
[7] Ramsay et al. (2001) Lancet 357(9251):195-6
[8] Lindberg (1999) Vaccine 17 Suppl 2:S28-36
[9] Buttery & Moxon (2000) J R Coll Physicians Lond 34:163-8
[10] Ahmad & Chapnick (1999) Infect Dis Clin North Am 13:113-33, vii
[11] Goldblatt (1998) J. Med. Microbiol. 47:563-567
[12] EP-B-0 477 508
[13] US patent 5,306,492
[14] WO98/42721
[15] Dick et al. in Conjugate Vaccines (eds. Cruse et al.) Karger, Basel, 1989, Vol. 10, 48-114
[16] Hermanson Bioconjugate Techniques, Academic Press, San Diego CA (1996)
[17] Bethell G.S. et al., J. Biol. Chem., 1979, 254, 2572-4
[18] Hearn M.T.W., J. Chromatogr., 1981, 218, 509-18
[19] Mol. Immunol., 1985, 22, 907-919
[20] EP-A-0208375
[21] WO00/10599
[22] Gever et al., Med. Microbiol. Immunol, 165 : 171-288 (1979).
[23] US patent 4,057,685.
[24] US patents 4,673,574; 4,761,283; 4,808,700.
[25] US patent 4,459,286.
[26] US patent 4,965,338
[27] US patent 4,663,160.
*[28] Research Disclosure,* 453077 (Jan 2002)
[29] EP-A-0372501
[30] EP-A-0378881
[31] EP-A-0427347
[32] WO93/17712
[33] WO94/03208

[34] WO98/58668
[35] EP-A-0471177
[36] WO00/56360
[37] WO91/01146
[38] WO00/61761
[39] WO01/72337
[40] Lei et al. (2000) Dev Biol (Basel) 103:259-264
[41] WO00/38711
[42] WO99/42130
[43] WO96/40242
[44] WO00/56365
[45] WO98/20734
[46] Vaccine design: the subunit and adjuvant approach, eds. Powell & Newman, Plenum Press 1995 (ISBN 0-306-44867-X).
[47] WO90/14837.
[48] WO00/07621.
[49]WO99/27960.
[50] European patent applications 0835318, 0735898 and 0761231.
[51] Krieg (2000) Vaccine 19:618-622; Krieg (2001) Curr opin Mol Ther 2001 3:15-24; WO96/02555, WO98/16247, WO98/18810, WO98/40100, WO98/55495, WO98/37919 and WO98/52581 *etc.*
[52] WO99/52549.
[53] WO01/21207.
[54] WO01/21152.
[55] WO00/62800.
[56] WO00/23105.
[57] WO99/11241.
[58] WO98/57659.
[59] Del Giudice et al. (1998) Molecular Aspects of Medicine, vol. 19, number 1.
[60] Johnson et al. (1999) Bioorg Med Chem Lett 9:2273-2278.
[61] International patent application WO00/50078.
[62] Singh et al. (2001) J. Cont. Rele. 70:267-276.
[63]International patent application WO 03/007985.
[64] Covacci & Rappuoli (2000) J. Exp. Med. 19:587-592.
[65] WO93/18150.
[66] Covacci et al. (1993) Proc. Natl. Acad. Sci. USA 90: 5791-5795.
[67] Tummuru et al. (1994) Infect. Immun. 61:1799-1809.
[68] Marchetti et al. (1998) Vaccine 16:33-37.
[69] Telford et al. (1994) J. Exp. Med. 179:1653-1658.
[70] Evans et al. (1995) Gene 153:123-127.
[71] WO96/01272 & WO96/01273, especially SEQ ID NO:6.
[72] WO97/25429.
[73] WO98/04702.
[74] WO99/24578.
[75] WO99/36544.
[76] WO99/57280.
[77] WO00/22430.
[78] Tettelin et al. (2000) Science 287:1809-1815.
[79] WO96/29412.
[80] Pizza et al. (2000) Science 287:1816-1820.
[81] WO01/52885.
[82] Bjune et al. (1991) Lancet 338(8775):1093-1096.
[83] Fukasawa et al. (1999) Vaccine 17:2951-2958.
[84] Rosenqvist et al. (1998) Dev. Biol. Stand. 92:323-333.
[85] Costantino et al. (1992) Vaccine 10:691-698.
[86] Costantino et al. (1999) Vaccine 17:1251-1263.
[87] Watson (2000) Pediatr Infect Dis J 19:331-332.
[88] Rubin (2000) Pediatr Clin North Am 47:269-285, v.
[89] Jedrzejas (2001) Microbiol Mol Biol Rev 65:187-207.

[90] Bell (2000) Pediatr Infect Dis J 19:1187-1188.
[91] Iwarson (1995) APMIS 103:321-326.
[92] Gerlich et al. (1990) Vaccine 8 Suppl:S63-68 & 79-80.
[93] Hsu et al. (1999) Clin Liver Dis 3:901-915.
[94] Gustafsson et al. (1996) N. Engl. J. Med. 334:349-355.
[95] Rappuoli et al. (1991) TIBTECH 9:232-238.
[96] Vaccines (1988) eds. Plotkin & Mortimer. ISBN 0-7216-1946-0.
[97] Del Guidice et al. (1998) Molecular Aspects of Medicine 19:1-70.
[98] WO02/02606.
[99] Kalman et al. (1999) Nature Genetics 21:385-389.
[100] Read et al. (2000) Nucleic Acids Res 28:1397-406.
[101] Shirai et al. (2000) J. Infect. Dis. 181 (Suppl 3):S524-S527.
[102] WO99/27105.
[103] WO00/27994.
[104] WO00/37494.
[105] WO99/28475.
[106] Ross et al. (2001) Vaccine 19:4135-4142.
[107] Sutter et al. (2000) Pediatr Clin North Am 47:287-308.
[108] Zimmerman & Spann (1999) Am Fam Physician 59:113-118, 125-126.
[109] Dreesen (1997) Vaccine 15 Suppl: S2-6.
[110] MMWR Morb Mortal Wkly Rep 1998 Jan 16;47(1):12, 19.
[111] McMichael (2000) Vaccine 19 Suppl 1:S101-107.
[112] Schuchat (1999) Lancet 353(9146):51-6.
[113] WO02/34771.
[114] Dale (1999) Infect Dis Clin North Am 13:227-43, viii.
[115] Ferretti et al. (2001) PNAS USA 98: 4658-4663.
[116] Kuroda et al. (2001) Lancet 357(9264):1225-1240; see also pages 1218-1219.
[117] J Toxicol Clin Toxicol (2001) 39:85-100.
[118] Demicheli et al. (1998) Vaccine 16:880-884.
[119] Stepanov et al. (1996) J Biotechnol 44:155-160.
[120] Ingram (2001) Trends Neurosci 24:305-307.
[121] Rosenberg (2001) Nature 411:380-384.
[122] Moingeon (2001) Vaccine 19:1305-1326.
[123] Robinson & Torres (1997) Seminars in Immunology 9:271-283.
[124] Donnelly et al. (1997) Annu Rev Immunol 15:617-648.
[125] Scott-Taylor & Dalgleish (2000) Expert Opin Investig Drugs 9:471-480.
[126] Apostolopoulos & Plebanski (2000) Curr Opin Mol Ther 2:441-447.
[127] Ilan (1999) Curr Opin Mol Ther 1:116-120.
[128] Dubensky et al. (2000) Mol Med 6:723-732.
[129] Robinson & Pertmer (2000) Adv Virus Res 55:1-74.
[130] Donnelly et al. (2000) Am J Respir Crit Care Med 162(4 Pt 2): S 190-193.
[131] Davis (1999) Mt. Sinai J. Med. 66:84-90.
[132] Chen et al., Anal. Chem., 1956, 28, 1756-8.
[133] Anderson et al., J. Clin. Invest., 1985, 76, 52-9
[134] Wolfenden R., J. Am. Chem. Soc., 1988, 120, 6814-5
[135] Habbeb et al. Anal. Biochem: (1966) 14: 328-336
[136] Miron & Wilchek (1982) Anal. Biochem. 126: 433-435
[137] Ricci et al. (2001) Vaccine 19:1989-1997.
[138] Carlone et al. (1992) J. Clin. Microbiol. 30:154-159.

## Claims

1. A modified capsular saccharide comprising a blocking group at a hydroxyl group position on at least one of the monosaccharide units of the corresponding native capsular saccharide, wherein the capsular saccharide comprises phosphodiester linkages.

2. The modified capsular saccharide of claim 1 wherein the at least one monosaccharide unit is a non-terminal monosaccharide unit.

**3.** The modified capsular saccharide of claim 1 or 2 which comprises at least one free hydroxyl group or amino group.

**4.** The modified capsular saccharide of claim 3 wherein the at least one free hydroxyl group is a terminal anomeric hydroxyl group.

**5.** The modified capsular saccharide of claim 1 wherein the blocking group is an electron-withdrawing group.

**6.** The modified capsular saccharide of claim 1 or claim 2 wherein the blocking group is of the formula:

$$-O-X-Y \text{ or } -OR^3$$

wherein
X is C(O), S(O) or $SO_2$;
Y is $C_{1-12}$ alkyl, $C_{1-12}$ alkoxy, $C_{3-12}$ cycloalkyl, $C_{5-12}$ aryl or $C_{5-12}$ aryl-$C_{1-6}$ alkyl, each of which may optionally be substituted with 1, 2 or 3 groups independently selected from F, Cl, Br, $CO_2H$, $CO_2(C_{1-6}$ alkyl), CN, $CF_3$ or $CCl_3$; or Y is $NR^1R^2$;
$R^1$ and $R^2$ are independently selected from H, $C_{1-12}$ alkyl, $C_{3-12}$ cycloalkyl, $C_{5-12}$ aryl, $C_{5-12}$ aryl-$C_{1-6}$ alkyl; or $R^1$ and $R^2$ may be joined to form a $C_{3-12}$ saturated heterocyclic group;
$R^3$ is $C_{1-12}$ alkyl or $C_{3-12}$ cycloalkyl, each of which may optionally be substituted with 1, 2 or 3 groups independently selected from F, Cl, Br, $CO_2(C_{1-6}$ alkyl), CN, $CF_3$ or $CCl_3$; or $R^3$ is $C_{5-12}$ aryl or $C_{5-12}$ aryl-$C_{1-6}$ alkyl, each of which may optionally be substituted with 1, 2, 3, 4 or 5 groups selected from F, Cl, Br, $CO_2H$, $CO_2(C_{1-6}$ alkyl), CN, $CF_3$ or $CCl_3$,

**7.** The modified capsular saccharide of claim 6 wherein the blocking group is $-OC(O)NR^1R^2$ or $-OC(O)CF_3$.

**8.** The modified capsular saccharide of claim 7 wherein the blocking group is $-OC(O)NR^1R^2$, and $R^1$ and $R^2$ are independently selected from $C_{1-6}$ alkyl.

**9.** The modified capsular saccharide of claim 8 wherein $R^1$ and $R^2$ are both methyl.

**10.** The modified capsular saccharide of any preceding claim wherein at least 10% of the monosaccharide units comprises a blocking group.

**11.** The modified capsular saccharide of any preceding claim, wherein the corresponding capsular saccharide comprises monosaccharide units linked by phosphodiester bonds.

**12.** The modified capsular saccharide of claim 11, wherein the corresponding capsular saccharide is a *Neisseria meningitidis* serogroup A saccharide.

**13.** The modified capsular saccharide of claim 12 wherein the blocking group is at any of the 4- and/or 3-positions of the corresponding *Neisseria meningitidis* serogroup A saccharide.

**14.** The modified capsular saccharide of claim 12 wherein the blocking group is at any of the 4-positions of the corresponding *Neisseria meningitidis* serogroup A saccharide.

**15.** The modified capsular saccharide of claims 1 to 14 which is an oligosaccharide.

**16.** A saccharide of the formula:

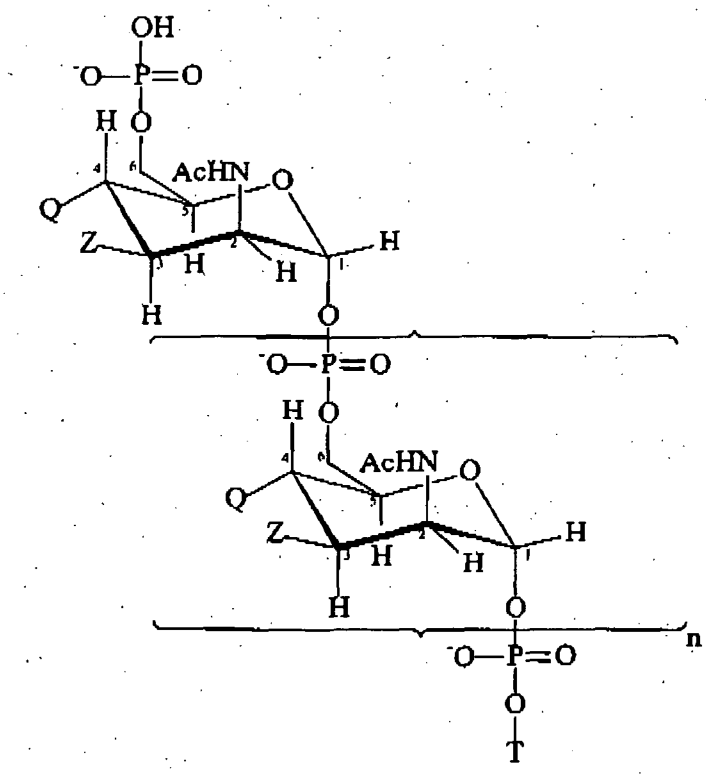

wherein
T is of the formula (A) or (B):

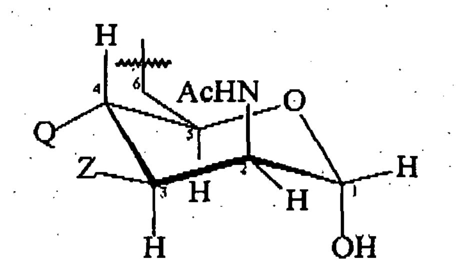

(A)

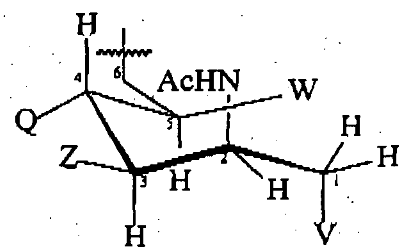

(B)

n is an integer from 1 to 100;
each Z group is independently selected from OH or a blocking group as defined in claims 5 to 9; and
each Q group is independently selected from OH or a blocking group as defined in claims 5 to 9;
W is selected from OH or a blocking group as defined in claims 5 to 9;
V is selected from $-NH_2$ or -NH-E, where E is a nitrogen protecting group;
and wherein more than about 7% of the Q groups are blocking groups.

**17.** The saccharide of claim 16 wherein at least 50% of the Z groups are OAc.

**18.** The saccharide of claims 16 or 17 wherein n is an integer from 15 to 25.

**19.** The saccharide of claims 16 to 18 wherein at least 10% of the Q groups are blocking groups.

**20.** A process for modifying a capsular saccharide comprising the steps of:

(a) providing a capsular saccharide having at least one hydroxyl group on a monosaccharide unit; and

(b) converting said at least one hydroxyl group into a blocking group;

wherein the capsular saccharide comprises phosphodiester linkages.

**21.** A process for modifying a capsular saccharide comprising the steps of:

(a) providing a capsular saccharide having at least one hydroxyl group on a monosaccharide unit;
(b) converting said at least one hydroxyl group into a blocking group $-OC(O)NR^1R^2$ by (b1) reacting the capsular saccharide with a bifunctional reagent in an organic solvent; and (b2) reacting the product of step (b1) with an amino compound of formula (I):

$$HNR^1R^2 \qquad (I)$$

wherein $R^1$ and $R^2$ are as defined in any of claims 3, 5 and 6;
wherein the capsular saccharide comprises phosphodiester linkages.

**22.** The process of claim 20 or claim 21, wherein the blocking group is as defined in any of claims 5 to 9.

**23.** The process of claim 22, wherein the organic solvent is an aprotic solvent.

**24.** The process of claim 23 wherein the aprotic solvent is selected from dimethylsulfoxide (DMSO), dimethylformamide (DMF), formamide, hexamethylphosphoramide (HMPA), hexamethylphosphorus triamide (HMPT), 1,3-dimethyl-3,4,5,6-tetrahydro-2(1*H*)-pyrimidinone (DMPU) or dimethylacetamide (DMAC).

**25.** The process of claims 23 or 24 wherein the aprotic solvent is DMSO.

**26.** The process of claims 22 to 25 wherein the bifunctional reagent is selected from 1,1'-carbonyldiimidazole (CDI), carbonyl di-1,2,4-triazole (CDT), carbonyl di-1,2,3-benzotriazole (CDB), diphenylcarbonate, cyanogen bromide, phosgene or triphosgene.

**27.** The process of claim 26 wherein the bifunctional reagent is CDI.

**28.** The process of claims 20 to 27 wherein the modified capsular saccharide is a modified capsular oligosaccharide.

**29.** The process of claim 28 wherein the capsular saccharide in step (a) is a capsular oligosaccharide obtainable by sizing the corresponding native capsular polysaccharide.

**30.** The process of claim 28 wherein the capsular saccharide in step (a) is a native capsular polysaccharide and the process further comprises a step (c) in which the product of step (b) is sized, thereby providing a modified capsular oligosaccharide.

**31.** A process for modifying a *Neisseria meningitidis* serogroup A polysaccharide comprising the steps of:

(a) providing a native *Neisseria meningitidis* serogroup A polysaccharide,
(b) sizing said polysaccharide to provide an oligosaccharide; and
(c) converting at least one hydroxyl group of the oligosaccharide into a blocking group, in accordance with any of claims 21 to 27.

**32.** A process for modifying a *Neisseria meningitidis* serogroup A polysaccharide comprising the steps of:

(a) providing a native *Neisseria meningitidis* serogroup A polysaccharide;
(b) converting at least one hydroxyl group of the polysaccharide into a blocking group, in accordance with any of claims 21 to 27; and
(c) sizing the resulting polysaccharide.

**33.** A process for preparing the modified capsular saccharide of claims 1 to 19 which is a total synthesis process comprising forming glycosidic linkages between two or more monosaccharide units.

**34.** A modified capsular saccharide obtainable by the process of any of claims 20 to 33.

**35.** A modified capsular saccharide obtained by the process of any of claims 20 to 33.

**36.** A saccharide-protein conjugate of a modified saccharide according to any one of claims 1 to 19, 34 or 35.

**37.** The conjugate of claim 36, wherein the protein is a bacterial toxin or toxoid.

**38.** The conjugate of claim 37, wherein the bacterial toxin or toxoid is diphtheria toxin or toxoid.

**39.** The conjugate of claim 37, wherein the bacterial toxin or toxoid is $CRM_{197}$.

**40.** A molecule comprising a saccharide moiety of formula:

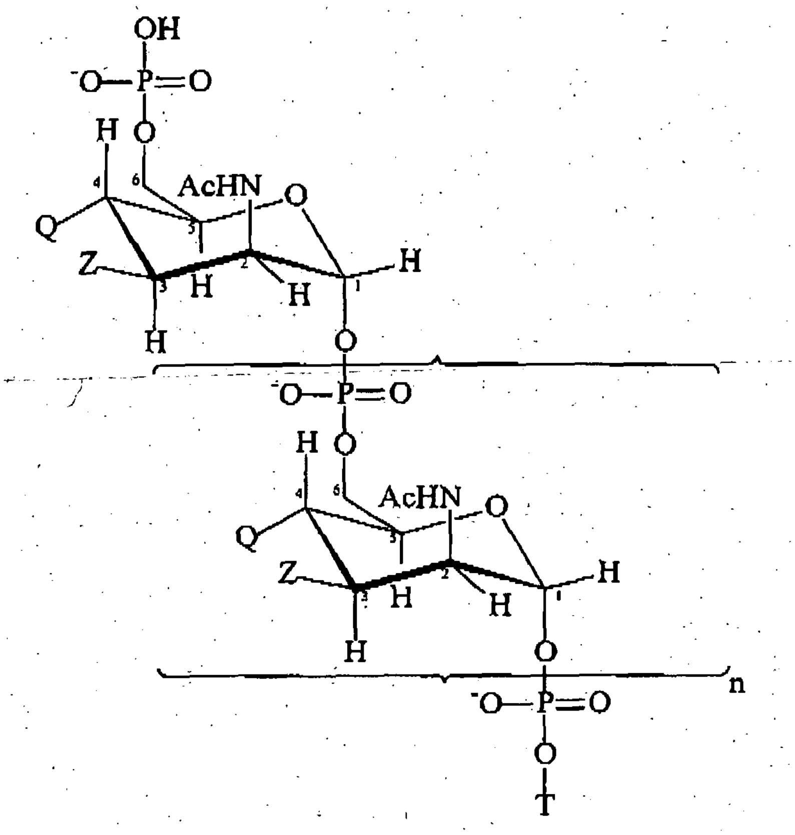

wherein
T is of the formula (A) or (B):

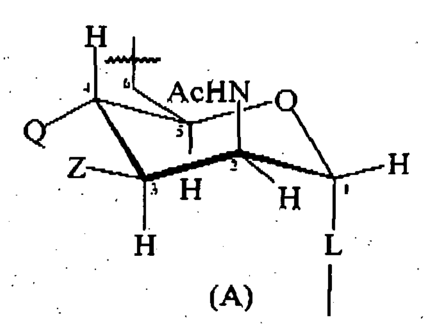

(A)

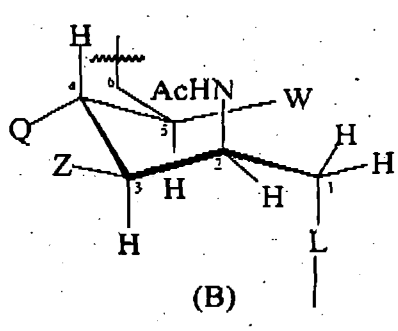

(B)

n is an integer from 1 to 100;

each Z group is independently selected from OH or a blocking group as defined in claims 5 to 9; and
each Q group is independently selected from OH or a blocking group as defined in claims 5 to 9;
W is selected from OH or a blocking group as defined in claims 5 to 9;
L is O, NH, NE, S or Se, where E is a nitrogen protecting group;
and wherein more than about 7% of the Q groups are blocking groups.

**41.** A pharmaceutical composition comprising (a) a modified saccharide according to any one of claims 1 to 19, 34 or 35 and/or a saccharide-protein conjugate according to any one of claims 36 to 39 and/or a molecule according to claim 40, and (b) a pharmaceutically acceptable carrier.

**42.** The composition of claim 41, further comprising a saccharide antigen from one or more of serogroups C, W135 and Y of *N. meningitidis,* the saccharide optionally being an oligosaccharide and optionally being conjugated to a carrier protein.

**43.** The composition of claim 41 or claim 42, further comprising a vaccine adjuvant.

**44.** The composition of claim 43, wherein the adjuvant is an aluminium phosphate.

**45.** The composition of any one of claims 41 to 44, which is a vaccine against a disease caused by Neisseria meningitidis.

**46.** The modified saccharide of any one of claims 1 to 18 for use as a medicament.

**47.** The conjugate of any one of claims 36 to 39 for use as a medicament.

**48.** The molecule of claim 40 for use as a medicament.

**49.** The use of the modified polysaccharide of any one of claims 1 to 19, 34 or 35, or the conjugate of any one of claims 36 to 39, or the molecule according to claims 40, in the manufacture of a medicament for preventing or treating a disease caused by one or more capsulate bacteria.

**50.** The use of claim 49, wherein the disease is bacterial meningitis.

**51.** The modified capsular saccharide of claim 6 wherein X is C(O).

**52.** The modified capsular saccharide of claim 6 wherein Y is $C_{1-12}$ alkyl.

**Patentansprüche**

**1.** Modifiziertes Kapselsaccharid, umfassend eine blockierende Gruppe an einer Hydroxylgruppenposition an mindestens einer der Monosaccharideinheiten des entsprechenden natürlich vorkommenden Kapselsaccharids, wobei das Kapselsaccharid Phosphordiesterverknüpfungen umfasst.

**2.** Modifiziertes Kapselsaccharid nach Anspruch 1, wobei die mindestens eine Monosaccharideinheit eine nicht-terminale Monosaccharideinheit ist.

**3.** Modifiziertes Kapselsaccharid nach Anspruch 1 oder 2, welches mindestens eine freie Hydroxyl- oder Aminogruppe umfasst.

**4.** Modifiziertes Kapselsaccharid nach Anspruch 3, wobei die mindestens eine freie Hydroxylgruppe eine terminale anomere Hydroxylgruppe ist.

**5.** Modifiziertes Kapselsaccharid nach Anspruch 1, wobei die blockierende Gruppe eine elektronenziehende Gruppe ist.

**6.** Modifiziertes Kapselsaccharid nach Anspruch 1 oder 2, wobei die blockierende Gruppe die Formel:

$$-O-X-Y \text{ oder } -OR^3$$

hat, wobei
X C(O), S(O) oder $SO_2$ ist,
Y $C_{1-12}$-Alkyl, $C_{1-12}$-Alkoxy, $C_{3-12}$-Cycloalkyl, $C_{5-12}$-Aryl oder $C_{5-12}$-Aryl-$C_{1-6}$-alkyl ist, von denen jedes gegebenenfalls mit 1, 2 oder 3 unabhängig aus F, Cl, Br, $CO_2H$, $CO_2(C_{1-6}$-Alkyl), CN, $CF_3$ oder $CCl_3$ ausgewählten Gruppen substituiert sein kann, oder Y ist $NR^1R^2$;
$R^1$ und $R^2$ unabhängig ausgewählt sind aus H, $C_{1-12}$-Alkyl, $C_{3-12}$-Cycloalkyl, $C_{5-12}$-Aryl, $C_{5-12}$-Aryl-$C_{1-6}$-alkyl; oder $R^1$ und $R^2$ verbunden sein können um einen gesättigten heterocyclischen $C_{3-12}$-Rest zu bilden;
$R^3$ $C_{1-12}$-Alkyl oder $C_{3-12}$-Cycloalkyl ist, von denen jedes gegebenenfalls mit 1, 2 oder 3 unabhängig aus F, Cl, Br, $CO_2(C_{1-6}$-Alkyl), CN, $CF_3$ oder $CCl_3$ ausgewählten Gruppen substituiert sein kann, oder $R^3$ ist $C_{5-12}$-Aryl oder $C_{5-12}$-Aryl-$C_{1-6}$-alkyl, von denen jedes gegebenenfalls mit 1, 2, 3, 4 oder 5 aus F, Cl, Br, $CO_2H$, $CO_2(C_{1-6}$-Alkyl), CN, $CF_3$ oder $CCl_3$ ausgewählten Gruppen substituiert sein kann.

**7.** Modifiziertes Kapselsaccharid nach Anspruch 6, wobei die blockierende Gruppe $-OC(O)NR^1R^2$ oder $-OC(O)CF_3$ ist.

**8.** Modifiziertes Kapselsaccharid nach Anspruch 7, wobei die blockierende Gruppe $-OC(O)NR^1R^2$ ist und $R^1$ und $R^2$ unabhängig ausgewählt sind aus $C_{1-6}$-Alkyl.

**9.** Modifiziertes Kapselsaccharid nach Anspruch 8, wobei $R^1$ und $R^2$ beide Methyl sind.

**10.** Modifiziertes Kapselsaccharid nach einem der vorausgehenden Ansprüche, wobei mindestens 10% der Monosaccharideinheiten eine blockierende Gruppe umfassen.

**11.** Modifiziertes Kapselsaccharid nach einem der vorausgehenden Ansprüche, wobei das entsprechende Kapselsaccharid über Phosphordiesterbindungen verknüpfte Monosaccharideinheiten umfasst.

**12.** Modifiziertes Kapselsaccharid nach Anspruch 11, wobei das entsprechende Kapselsaccharid ein *Neisseria meningitidis* Serumgruppen-A-Saccharid ist.

**13.** Modifiziertes Kapselsaccharid nach Anspruch 12, wobei die blockierende Gruppe an irgendeiner der 4- und/oder 3-Positionen des entsprechenden *Neisseria meningitidis* Serumgruppen-A-Saccharids ist.

**14.** Modifiziertes Kapselsaccharid nach Anspruch 12, wobei die blockierende Gruppe an irgendeiner der 4-Positionen des entsprechenden *Neisseria meningitidis* Serumgruppen-A-Saccharids ist.

**15.** Modifiziertes Kapselsaccharid nach Ansprüchen 1 bis 14 welches ein Oligosaccharid ist.

**16.** Saccharid der Formel:

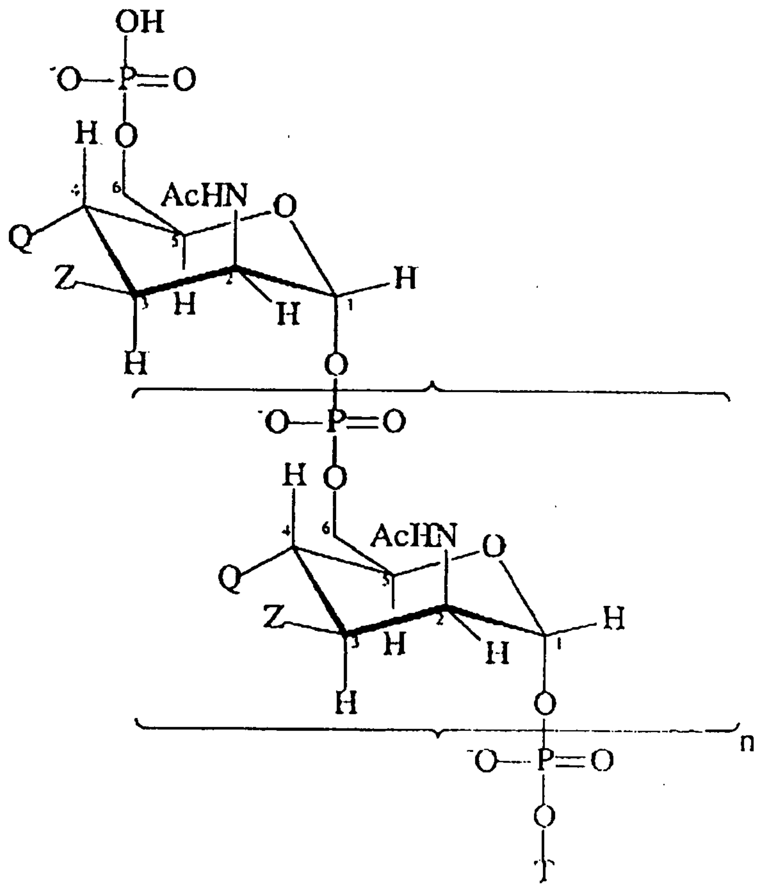

wobei

T die Formel (A) oder (B) hat:

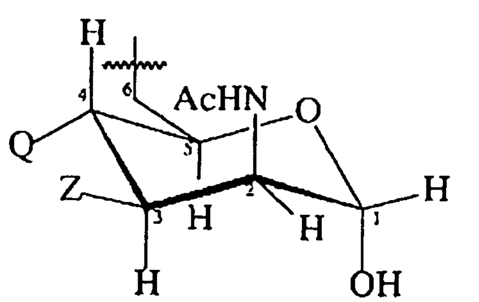

(A)

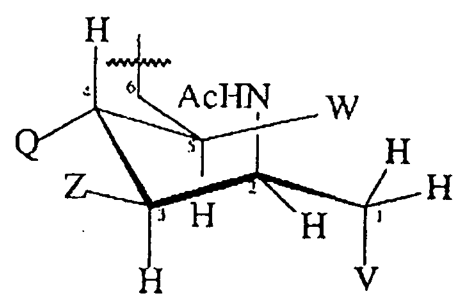

(B)

n eine ganze Zahl von 1 bis 100 ist;

jeder Rest Z unabhängig ausgewählt ist aus OH oder einer blockierenden Gruppe wie in den Ansprüchen 5 bis 9 definiert; und

jeder Rest Q unabhängig ausgewählt ist aus OH oder einer blockierenden Gruppe wie in den Ansprüchen 5 bis 9 definiert;

W ausgewählt ist aus OH oder einer blockierenden Gruppe wie in den Ansprüchen 5 bis 9 definiert;

V ausgewählt ist aus $-NH_2$ oder -NH-E, wobei E eine Stickstoffschutzgruppe ist;

und wobei mehr als etwa 7% der Reste Q blockierende Gruppen sind.

**17.** Saccharid nach Anspruch 16, wobei mindestens 50% der Reste Z OAc sind.

**18.** Saccharid nach Anspruch 16 oder 17, wobei n eine ganze Zahl von 15 bis 25 ist.

**19.** Saccharid nach Ansprüchen 16 bis 18, wobei mindestens 10% der Reste Q blockierende Gruppen sind.

**20.** Verfahren zur Modifizierung eines Kapselsaccharids umfassend die Schritte:

(a) Bereitstellung eines Kapselsaccharids mit mindestens einer Hydroxylgruppe an einer Monosaccharideinheit; und
(b) Umwandlung dieser mindestens einen Hydroxylgruppe in eine blockierende Gruppe;

wobei das Kapselsaccharid Phosphordiesterverknüpfungen umfasst.

**21.** Verfahren zur Modifizierung eines Kapselsaccharids umfassend die Schritte:

(a) Bereitstellung eines Kapselsaccharids mit mindestens einer Hydroxylgruppe an einer Monosaccharideinheit;
(b) Umwandlung dieser mindestens einen Hydroxylgruppe in eine blockierende Gruppe $-OC(O)NR^1R^2$ durch (b1) Reaktion des Kapselsaccharids mit einem bifunktionalen Reagens in einem organischen Lösungsmittel; und (b2) Reaktion des Produkts aus Schritt (b1) mit einer Aminoverbindung der Formel (I):

$$HNR^1R^2 \qquad (I),$$

wobei $R^1$ und $R^2$ wie in einem der Ansprüche 3, 5 und 6 definiert sind;

wobei das Kapselsaccharid Phosphordiesterverknüpfungen umfasst.

**22.** Verfahren nach Anspruch 20 oder Anspruch 21, wobei die blockierende Gruppe wie in einem der Ansprüche 5 bis 9 definiert ist.

**23.** Verfahren nach Anspruch 22, wobei das organische Lösungsmittel ein aprotisches Lösungsmittel ist.

**24.** Verfahren nach Anspruch 23, wobei das aprotische Lösungsmittel ausgewählt ist aus Dimethylsulfoxid (DMSO), Dimethylformamid (DMF), Formamid, Hexamethylphosphoramid (HMPA), Hexamethylphosphortriamid (HMPT), 1,3-Dimethyl-3,4,5,6-tetrahydro-2(1*H*)-pyrimidinon (DMPU) oder Dimethylacetamid (DMAC).

**25.** Verfahren nach den Ansprüchen 23 oder 24, wobei das aprotische Lösungsmittel DMSO ist.

**26.** Verfahren nach den Ansprüchen 22 bis 25, wobei das bifunktionale Reagens ausgewählt ist aus 1,1'-Carbonyldiimidazol (CDI), Carbonyldi-1,2,4-triazol (CDT), Carbonyldi-1,2,3-benzotriazol (CDB), Diphenylcarbonat, Bromcyan, Phosgen oder Triphosgen.

**27.** Verfahren nach Anspruch 26, wobei das bifunktionale Reagens CDI ist.

**28.** Verfahren nach Ansprüchen 20 bis 27, wobei das modifizierte Kapselsaccharid ein modifiziertes Kapseloligosaccharid ist.

**29.** Verfahren nach Anspruch 28, wobei das Kapselsaccharid in Schritt (a) ein Kapseloligosaccharid ist, welches durch Größenausschlussbestimmung des entsprechenden natürlich vorkommenden Kapselsaccharids erhalten wird.

**30.** Verfahren nach Anspruch 28, wobei das Kapselsaccharid in Schritt (a) ein natürlich vorkommendes Kapselpolysaccharid ist und das Verfahren einen weiteren Schritt (c) umfasst, in welchem das Produkt aus Schritt (b) einer Größenausschlussbestimmung unterzogen wird, wodurch ein modifiziertes Kapseloligosaccharid erhalten wird.

**31.** Verfahren zur Modifizierung eines *Neisseria meningitidis* Serumgruppen-A-Polysaccharids umfassend die Schritte:

(a) Bereitstellung eines natürlich vorkommenden *Neisseria meningitidis* Serumgruppen-A-Polysaccharids;
(b) Größenausschlussbestimmung des Polysaccharids, um ein Oligosaccharid bereitzustellen; und
(c) Umwandlung mindestens einer Hydroxylgruppe des Ologosaccarids in eine blockierende Gruppe, in Übereinstimmung mit einem der Ansprüche 21 bis 27.

**32.** Verfahren zur Modifizierung eines *Neisseria meningitidis* Serumgruppen-A-Polysaccharids, umfassend die Schritte:

(a) Bereitstellung eines natürlich vorkommenden *Neisseria meningitidis* Serumgruppen-A-Polysaccharids;
(b) Umwandlung mindestens einer Hydroxylgruppe des Polysaccharids in eine blockierende Gruppe, in Übereinstimmung mit einem der Anspüche 21 bis 27; und
(c) Größenausschlussbestimmung des resultierenden Polysaccharids.

**33.** Verfahren zur Erzeugung des modifizierten Kapselsaccharids nach Anspruch 1 bis 19, welches ein Totalsyntheseprozess ist, welcher die Bildung von Glykosidbrücken zwischen zwei oder mehreren Monosaccharideinheiten umfasst.

**34.** Modifiziertes Kapselsaccharid, erhältlich durch das Verfahren nach einem der Ansprüche 20 bis 33.

**35.** Modifiziertes Kapselsaccharid, erhalten durch das Verfahren nach einem der Ansprüche 20 bis 33.

**36.** Saccharid-Protein-Konjugat eines modifizierten Saccharids gemäß einem der Ansprüche 1 bis 19, 34 oder 35.

**37.** Konjungat nach Anspruch 36, wobei das Protein ein bakteriales Toxin oder Toxoid ist.

**38.** Konjungat nach Anspruch 37, wobei das bakteriale Toxin oder Toxoid Diphtherietoxin oder -toxoid ist.

**39.** Konjungat nach Anspruch 37, wobei das bakteriale Toxin oder Toxoid $CRM_{197}$ ist.

**40.** Molekül, umfassend eine Saccharideinheit der Formel:

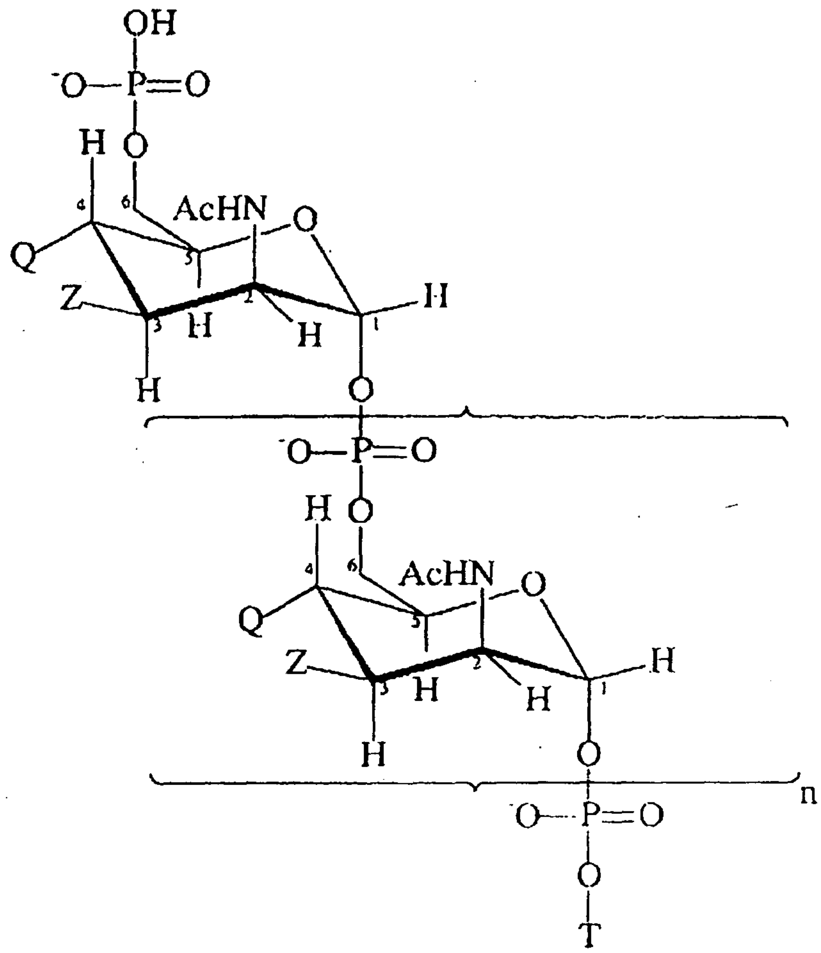

wobei

T die Formel (A) oder (B) hat:

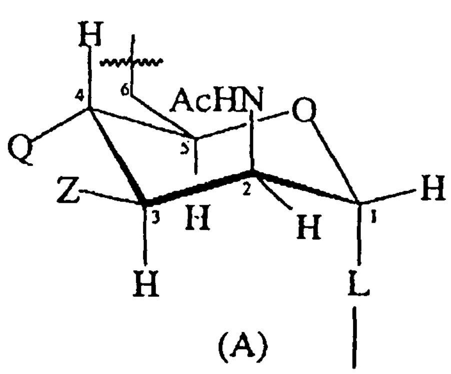

(A)

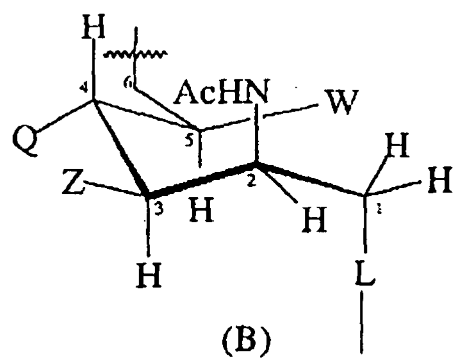

(B)

n eine ganze Zahl von 1 bis 100 ist;

jeder Rest Z unabhängig ausgewählt ist aus OH oder einer blockierenden Gruppe wie in den Ansprüchen 5 bis 9 definiert; und

jeder Rest Q unabhängig ausgewählt ist aus OH oder einer blockierenden Gruppe wie in den Ansprüchen 5 bis 9 definiert;

W ausgewählt ist aus OH oder einer blockierenden Gruppe wie in den Ansprüchen 5 bis 9 definiert;

L ausgewählt ist aus O, NH, NE, S, oder SE, wobei E eine Stickstoffschutzgruppe ist;
und wobei mehr als 7% der Reste Q blockierende Gruppen sind.

**41.** Arzneimittel, umfassend (a) ein modifiziertes Saccharid gemäß einem der Ansprüche 1 bis 19, 34 oder 35 und/oder ein Saccharid-Protein-Konjugat gemäß einem der Ansprüche 36 bis 39 und/oder ein Molekül gemäß Anspruch 40 und (b) einen pharmazeutisch verträglichen Träger.

**42.** Verbindung nach Anspruch 41, weiter umfassend ein Saccharidantigen von einem oder mehreren Serumgruppen C, W135 und Y von *N.meningitidis,* wobei das Saccharid gegebenenfalls ein Oligosaccharid sein und gegebenenfalls an ein Transportprotein konjugiert sein kann.

**43.** Verbindung nach Anspruch 41 oder Anspruch 42, weiter umfassend einen Impfhilfsstoff.

**44.** Verbindung nach Anspruch 43, wobei der Hilfsstoff ein Aluminiumphosphat ist.

**45.** Verbindung nach einem der Ansprüche 41 bis 44, welcher ein Impfstoff gegen eine durch Neisseria meningitidis ausgelöste Erkrankung ist.

**46.** Modifiziertes Saccharid nach einem der Ansprüche 1 bis 18 zur Verwendung als ein Medikament.

**47.** Konjugat nach einem der Ansprüche 36 bis 39 zur Verwendung als ein Medikament.

**48.** Molekül nach Anspruch 40 zur Verwendung als ein Medikament.

**49.** Verwendung des modifizierten Polysaccharids nach einem der Ansprüche 1 bis 19, 34 oder 35, oder des Konjugats nach einem der Ansprüche 36 bis 39 , oder des Moleküls nach Anspruch 40 in der Herstellung eines Medikaments zur Vorbeugung oder Behandlung einer durch eine oder mehrere kapselförmige Bakterien verursachte Erkrankung.

**50.** Verwendung nach Anspruch 49, wobei die Krankheit bakterielle Meningitis ist.

**51.** Modifiziertes Kapselsaccharid nach Anspruch 6, wobei X C(O) ist.

**52.** Modifiziertes Kapselsaccharid nach Anspruch 6, wobei Y $C_{1-12}$-Alkyl ist.

## Revendications

**1.** Saccharide capsulaire modifié comprenant un groupe bloquant au niveau d'une position de groupe hydroxyle sur au moins un des motifs monosaccharide du saccharide capsulaire natif correspondant, dans lequel le saccharide capsulaire comprend des liaisons phosphodiester.

**2.** Saccharide capsulaire modifié selon la revendication 1, dans lequel le ou les motifs monosaccharides sont des motifs monosaccharides non terminaux.

**3.** Saccharide capsulaire modifié selon la revendication 1 ou 2, qui comprend au moins un groupe amino ou hydroxyle libre.

**4.** Saccharide capsulaire modifié selon la revendication 3, dans lequel le ou les groupes hydroxyles libres sont des groupes hydroxyles anomère terminaux.

**5.** Saccharide capsulaire modifié selon la revendication 1, dans lequel le groupe bloquant est un groupe attracteur d'électron.

**6.** Saccharide capsulaire modifié selon la revendication 1 ou 2, dans lequel le groupe bloquant est de formule :

$$-O-X-Y \text{ ou } -OR^3,$$

dans lesquelles

X représente C(O), S(O) ou $SO_2$ ;
Y représente un groupe alkyle en $C_1$ à $C_{12}$, alcoxyle en $C_1$ à $C_{12}$, cycloalkyle en $C_3$ à $C_{12}$, aryle en $C_5$ à $C_{12}$ ou (aryle en $C_5$ à $C_{12}$)-(alkyle en $C_1$ à $C_6$), chacun pouvant éventuellement être substitué par 1, 2 ou 3 groupes indépendamment choisis parmi F, Cl, Br, $CO_2H$, $CO_2$(alkyle en $C_1$ à $C_6$), CN, $CF_3$ ou $CCl_3$ ; ou Y représente $NR^1R^2$ ;
$R^1$ et $R^2$ sont indépendamment choisis parmi un atome d'hydrogène, les groupes alkyle en $C_1$ à $C_{12}$, cycloalkyle en $C_3$ à $C_{12}$, aryle en $C_5$ à $C_{12}$, (aryle en $C_5$ à $C_{12}$) - (alkyle en $C_1$ à $C_6$) ; ; ou $R^1$ et $R^2$ peuvent être reliés pour former un groupe hétérocyclique saturé en $C_3$ à $C_{12}$ ;
$R^3$ représente un groupe alkyle en $C_1$ à $C_{12}$ ou cycloalkyle en $C_3$ à $C_{12}$, chacun pouvant éventuellement être substitué par 1, 2 ou 3 groupes indépendamment choisis parmi F, Cl, Br, $CO_2$ (alkyle en $C_1$ à $C_6$), CN, $CF_3$ ou $CCl_3$ ; ou $R^3$ représente un groupe aryle en $C_5$ à $C_{12}$ ou (aryle en $C_5$ à $C_{12}$)-(alkyle en $C_1$ à $C_6$), chacun pouvant éventuellement être substitué par 1, 2, 3, 4 ou 5 groupes choisis parmi F, Cl, Br, $CO_2H$, $CO_2$-(alkyle en $C_1$ à $C_6$), CN, $CF_3$ ou $CCl_3$.

**7.** Saccharide capsulaire modifié selon la revendication 6, dans lequel le groupe bloquant est -OC(O)$NR^1R^2$ ou -OC (O)$CF_3$.

**8.** Saccharide capsulaire modifié selon la revendication 7, dans lequel le groupe bloquant est -OC(O)$NR^1R^2$, et $R^1$ et $R^2$ sont indépendamment choisis parmi les groupes alkyle en $C_1$ à $C_6$.

**9.** Saccharide capsulaire modifié selon la revendication 8, dans lequel $R^1$ et $R^2$ représentent tous deux des groupes méthyle.

**10.** Saccharide capsulaire modifié selon l'une quelconque des revendications précédentes, dans lequel au moins 10% des motifs monosaccharide comprennent un groupe bloquant.

**11.** Saccharide capsulaire modifié selon l'une quelconque des revendications précédentes, dans lequel le saccharide capsulaire correspondant comprend des motifs monosaccharide liés par liaisons phosphodiester.

**12.** Saccharide capsulaire modifié selon la revendication 11, dans lequel le saccharide capsulaire correspondant est un saccharide de *Neisseria meningitidis* sérogroupe A.

**13.** Saccharide capsulaire modifié selon la revendication 12, dans lequel le groupe bloquant se trouve au niveau de l'une quelconque des positions 4 et/ou 3 du saccharide de *Neisseria meningitidis* sérogroupe A correspondant.

**14.** Saccharide capsulaire modifié selon la revendication 12, dans lequel le groupe bloquant se trouve au niveau de l'une quelconque des positions 4 du saccharide de *Neisseria meningitidis* sérogroupe A correspondant.

**15.** Saccharide capsulaire modifié selon les revendications 1 à 14, qui est un oligosaccharide.

**16.** Saccharide de formule :

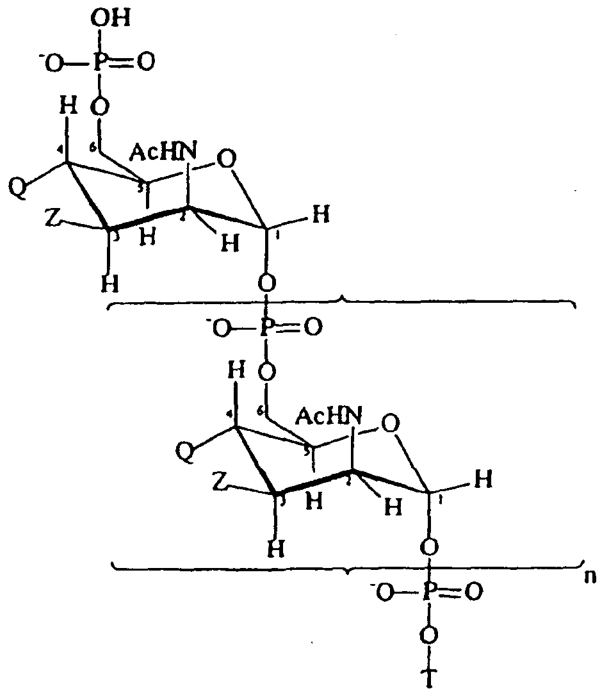

dans laquelle
T est de formule (A) ou (B) :

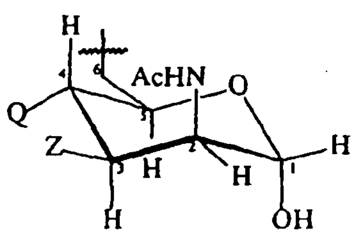

(A)

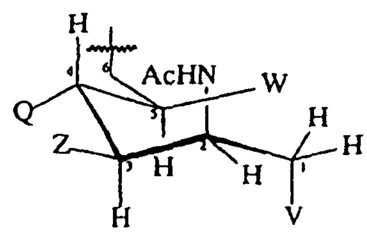

(B)

n est un nombre entier valant 1 à 100 ;
chaque groupe Z est indépendamment choisi parmi OH ou un groupe bloquant tel que défini dans les revendications 5 à 9 ; et
chaque groupe Q est indépendamment choisi parmi OH ou un groupe bloquant tel que défini dans les revendications 5 à 9 ;
W est choisi parmi OH ou un groupe bloquant tel que défini dans les revendications 5 à 9 ;
V est choisi parmi $-NH_2$ ou -NH-E, où E représente un groupe de protection azoté ;
et dans laquelle plus d'environ 7% des groupes Q sont des groupes bloquants.

**17.** Saccharide selon la revendication 16, dans lequel au moins 50% des groupes Z sont OAc.

**18.** Saccharide selon la revendication 16 ou 17, dans lequel n est un nombre entier valant 15 à 25.

**19.** Saccharide selon les revendications 16 à 18, dans lequel au moins 10% des groupes Q sont des groupes bloquants.

**20.** Procédé de modification d'un saccharide capsulaire comprenant les étapes consistant à :

(a) fournir un saccharide capsulaire comprenant au moins un groupe hydroxyle sur un motif monosaccharide ; et
(b) convertir ledit ou lesdits groupes hydroxyles en groupe bloquant ; dans lequel le saccharide capsulaire

comprend des liaisons phosphodiester.

**21.** Procédé de modification d'un saccharide capsulaire comprenant les étapes consistant à :

(a) fournir un saccharide capsulaire comprenant au moins un groupe hydroxyle sur un motif monosaccharide ;
(b) convertir ledit ou lesdits groupes hydroxyles en groupe bloquant $-OC(O)NR^1R^2$, en (b1) faisant réagir le saccharide capsulaire avec un réactif bifonctionnel dans un solvant organique ; et (b2) faire réagir le produit de l'étape (b1) avec un composé amino de formule (I) :

$$HNR^1R^2 \qquad (I)$$

dans laquelle $R^1$ et $R^2$ sont tels que définis dans l'une quelconque des revendications 3, 5 et 6 ;
dans lequel le saccharide capsulaire comprend des liaisons phosphodiester.

**22.** Procédé selon la revendication 20 ou 21, dans lequel le groupe bloquant est tel que défini dans l'une quelconque des revendications 5 à 9.

**23.** Procédé selon la revendication 22, dans lequel le solvant organique est un solvant aprotique.

**24.** Procédé selon la revendication 23, dans lequel le solvant aprotique est choisi parmi le diméthylsulfoxyde (DMSO), le diméthylformamide (DMF), le formamide, l'hexaméthylphosphoramide (HMPA), l'hexaméthylphosphorotriamide (HMPT), la 1,3-diméthyl-3,4,5,6-tétrahydro-2(1H)-pyrimidinone (DMPU) ou le diméthylacétamide (DMAC).

**25.** Procédé selon la revendication 23 ou 24, dans lequel le solvant aprotique est DMSO.

**26.** Procédé selon les revendications 22 à 25, dans lequel le réactif bifonctionnel est choisi parmi le 1,1'-carbonyldiimidazole (CDI), le carbonyl-di-1,2,4-triazole (CDT), le carbonyldi-1,2,3-benzotriazole (CDB), le diphényl-carbonate, le bromure de cyanogène, le phosgène ou le triphosgène.

**27.** Procédé selon la revendication 26, dans lequel le réactif bifonctionnel est CDI.

**28.** Procédé selon les revendications 20 à 27, dans lequel le saccharide capsulaire modifié est un oligosaccharide capsulaire modifié.

**29.** Procédé selon la revendication 28, dans lequel le saccharide capsulaire de l'étape (a) est un oligosaccharide capsulaire pouvant être obtenu en redimensionnant le polysaccharide capsulaire natif correspondant.

**30.** Procédé selon la revendication 28, dans lequel le saccharide capsulaire de l'étape (a) est un polysaccharide capsulaire natif et le procédé comprend en outre une étape (c) dans laquelle le produit de l'étape (b) est redimensionné, donnant ainsi un oligosaccharide capsulaire modifié.

**31.** Procédé de modification d'un polysaccharide de *Neisseria meningitidis* sérogroupe A comprenant les étapes consistant à :

(a) fournir un polysaccharide de *Neisseria meningitidis* sérogroupe A natif ;
(b) redimensionner ledit polysaccharide pour donner un oligosaccharide ; et
(c) convertir au moins un groupe hydroxyle de l'oligosaccharide en groupe bloquant, selon l'une quelconque des revendications 21 à 27.

**32.** Procédé de modification d'un polysaccharide de *Neisseria meningitidis* sérogroupe A comprenant les étapes consistant à :

(a) fournir un polysaccharide de *Neisseria meningitidis* sérogroupe A natif ;
(b) convertir au moins un groupe hydroxyle du polysaccharide en groupe bloquant, selon l'une quelconque des revendications 21 à 27 ; et
(c) redimensionner le polysaccharide obtenu.

**33.** Procédé de préparation du saccharide capsulaire modifié selon les revendications 1 à 19, qui est un procédé de synthèse totale consistant à former des liaisons glycosidiques entre deux motifs monosaccharide ou plus.

**34.** Saccharide capsulaire modifié pouvant être obtenu au moyen du procédé selon l'une quelconque des revendications 20 à 33.

**35.** Saccharide capsulaire modifié obtenu au moyen du procédé selon l'une quelconque des revendications 20 à 33.

**36.** Conjugué saccharide/protéine d'un saccharide modifié selon l'une quelconque des revendications 1 à 19, 34 ou 35.

**37.** Conjugué selon la revendication 36, dans lequel la protéine est une toxine ou une anatoxine bactérienne.

**38.** Conjugué selon la revendication 37, dans lequel la toxine ou l'anatoxine bactérienne est la toxine ou l'anatoxine diphtérique.

**39.** Conjugué selon la revendication 37, dans lequel la toxine ou l'anatoxine bactérienne est $CRM_{197}$.

**40.** Molécule comprenant une fraction saccharide de formule :

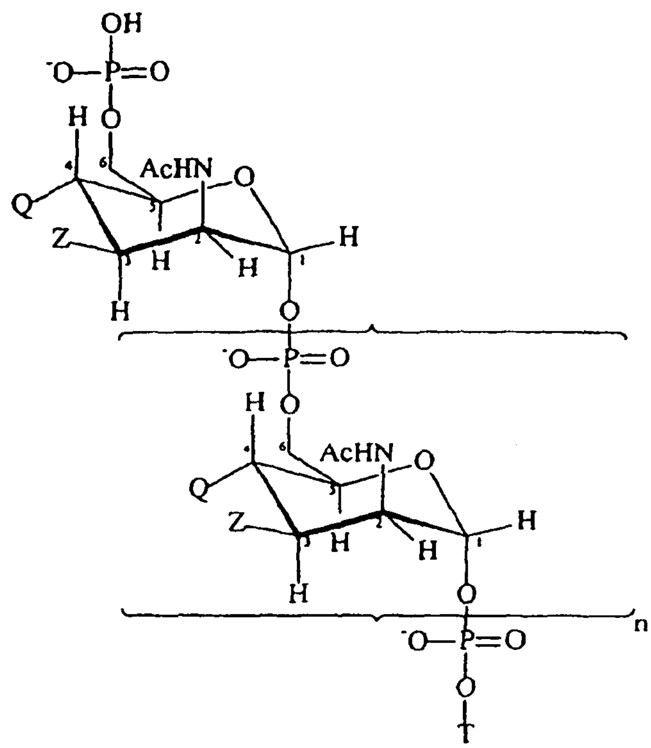

dans laquelle
T est de formule (A) ou (B) :

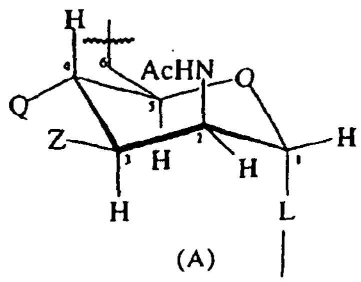

(A)

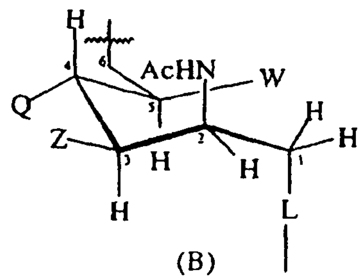

(B)

n est un nombre entier valant 1 à 100 ;
chaque groupe Z est indépendamment choisi parmi OH ou un groupe bloquant tel que défini dans les revendications 5 à 9 ; et
chaque groupe Q est indépendamment choisi parmi OH ou un groupe bloquant tel que défini dans les revendications 5 à 9 ;
W est choisi parmi OH ou un groupe bloquant tel que défini dans les revendications 5 à 9 ;
L représente O, NH, NE, S ou Se , où E représente un groupe de protection azoté ;
et dans laquelle plus d'environ 7% des groupes Q sont des groupes bloquants.

**41.** Composition pharmaceutique comprenant (a) un saccharide modifié selon l'une quelconque des revendications 1 à 19, 34 ou 35 et/ou un conjugué saccharide/protéine selon l'une quelconque des revendications 36 à 39 et/ou une molécule selon la revendication 40, et (b) un véhicule acceptable sur le plan pharmaceutique.

**42.** Composition selon la revendication 41, comprenant en outre un antigène saccharidique issu d'un ou plusieurs des sérogroupes C, W135 et Y de N. *meningitidis,* le saccharide étant éventuellement un oligosaccharide et étant éventuellement conjugué à une protéine porteuse.

**43.** Composition selon la revendication 41 ou 42, comprenant en outre un adjuvant de type vaccin.

**44.** Composition selon la revendication 43, dans laquelle l'adjuvant est un phosphate d'aluminium.

**45.** Composition selon l'une quelconque des revendications 41 à 44, qui est un vaccin contre une maladie provoquée par *Neisseria meningitidis.*

**46.** Saccharide modifié selon l'une quelconque des revendications 1 à 18, pour une utilisation en tant que médicament.

**47.** Conjugué selon l'une quelconque des revendications 36 à 39, pour une utilisation en tant que médicament.

**48.** Molécule selon la revendication 40, pour une utilisation en tant que médicament.

**49.** Utilisation du polysaccharide modifié selon l'une quelconque des revendications 1 à 19, 34 ou 35, ou du conjugué selon l'une quelconque des revendications 36 à 39, ou de la molécule selon la revendication 40, dans la fabrication d'un médicament pour prévenir ou traiter une maladie provoquée par une ou plusieurs bactéries encapsulées.

**50.** Utilisation selon la revendication 49, dans laquelle la maladie est la méningite bactérienne.

**51.** Saccharide capsulaire modifié selon la revendication 6, dans lequel X représente C(O).

**52.** Saccharide capsulaire modifié selon la revendication 6, dans lequel Y représente un groupe alkyle en $C_1$ à $C_{12}$.

*Figure 1*

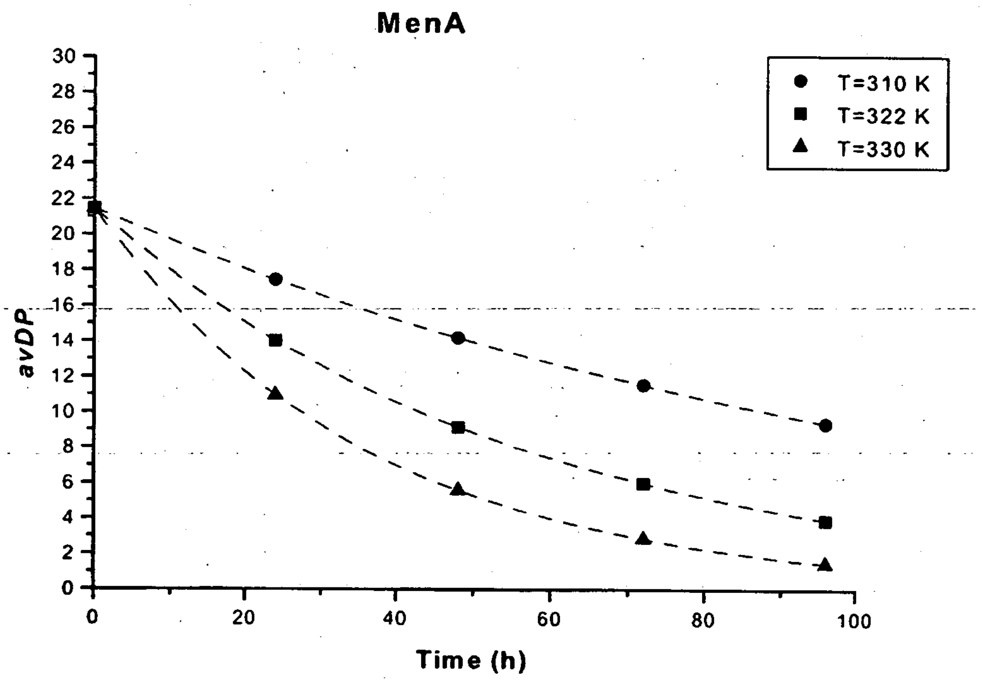


*Figure 2*

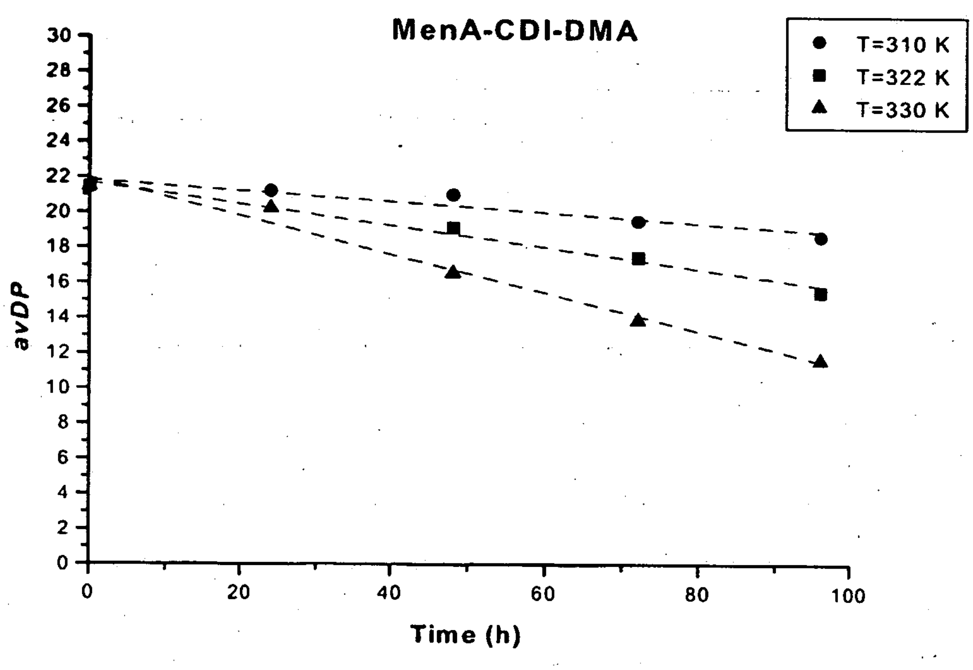

*Figure 3*

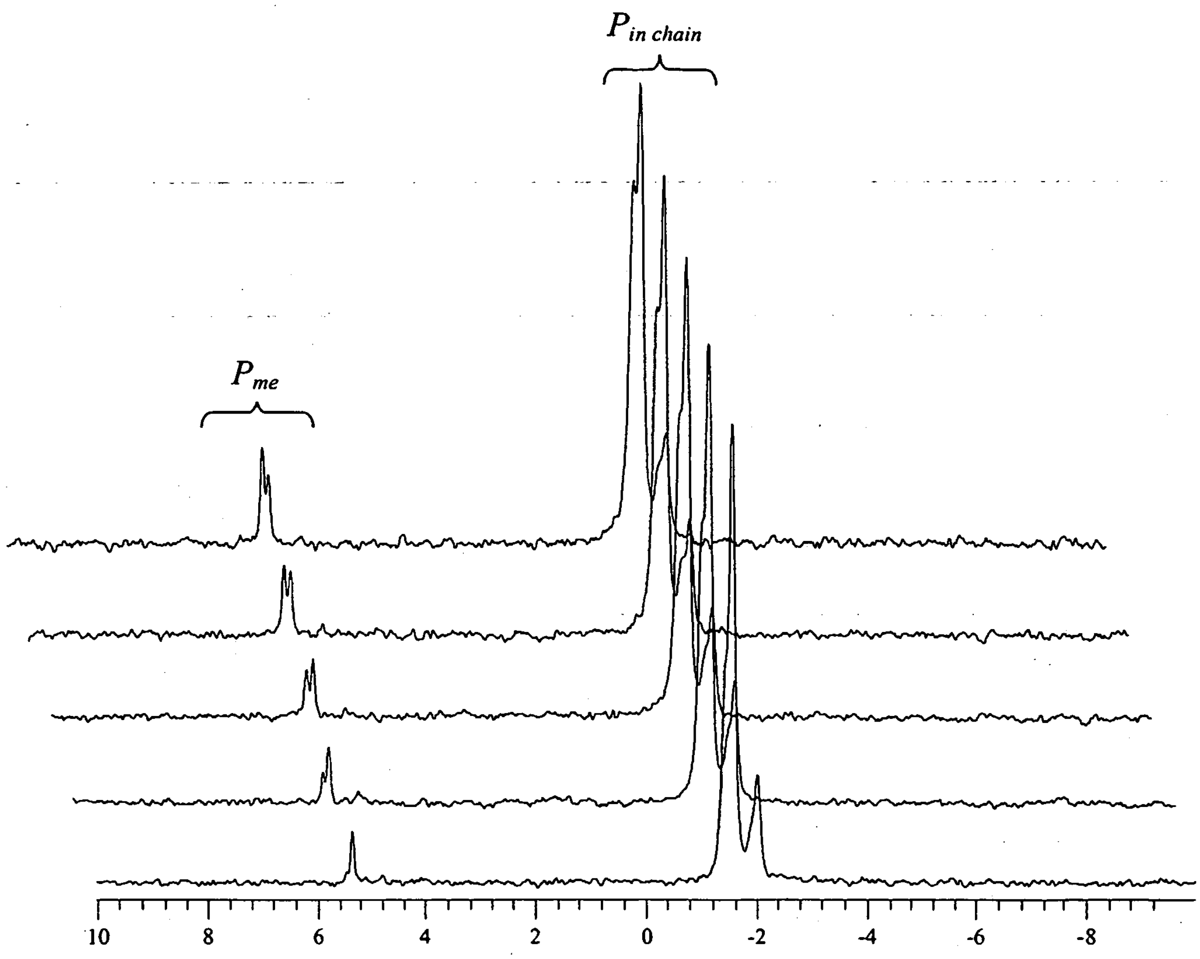

*Figure 4*

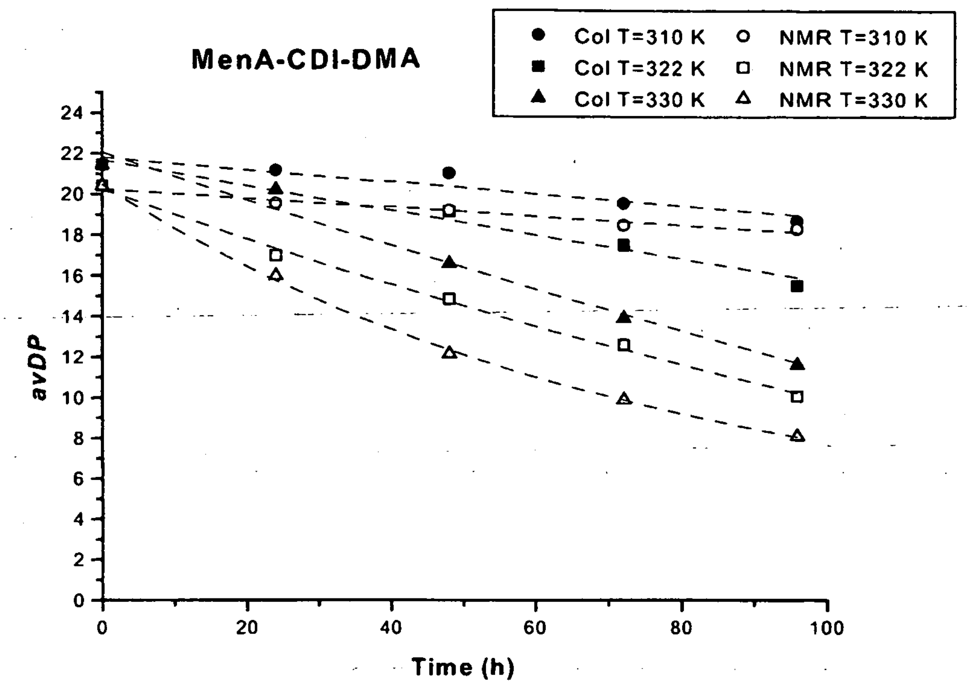


*Figure 5*

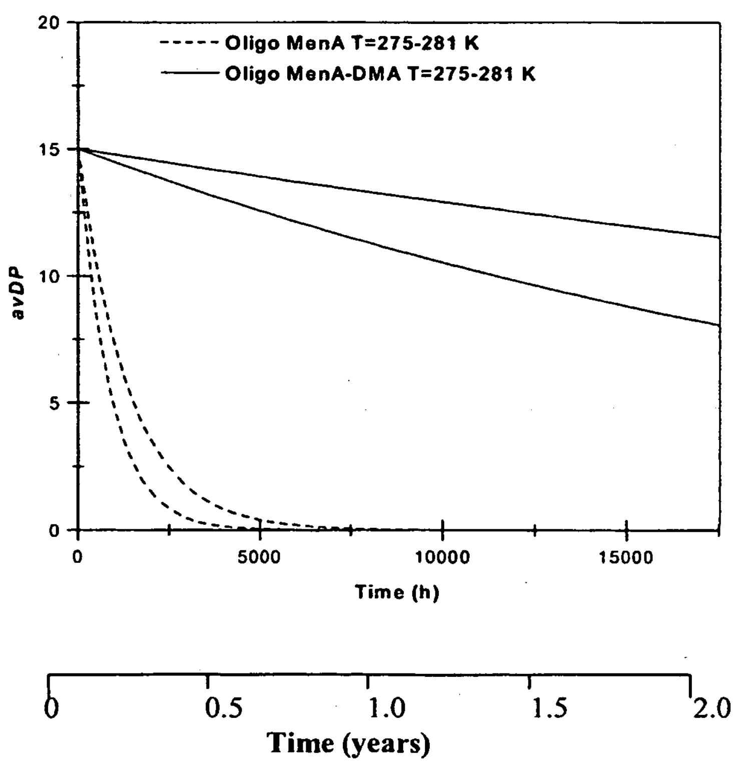

*Figure 6*

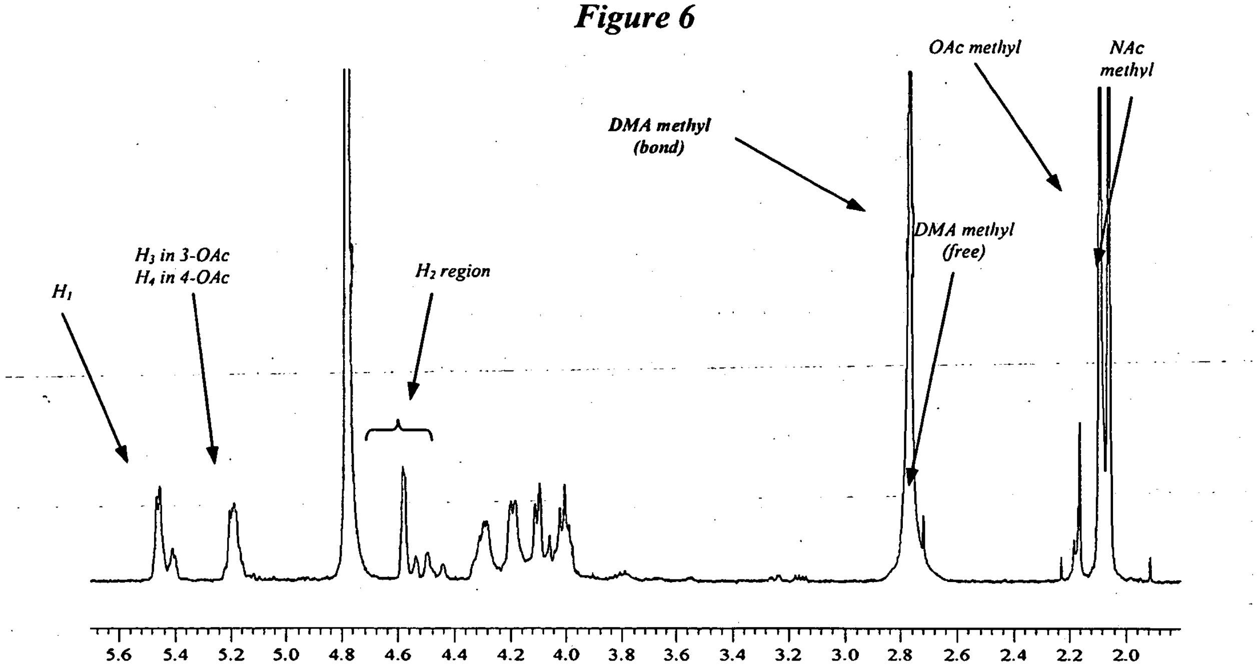


*Figure 7*

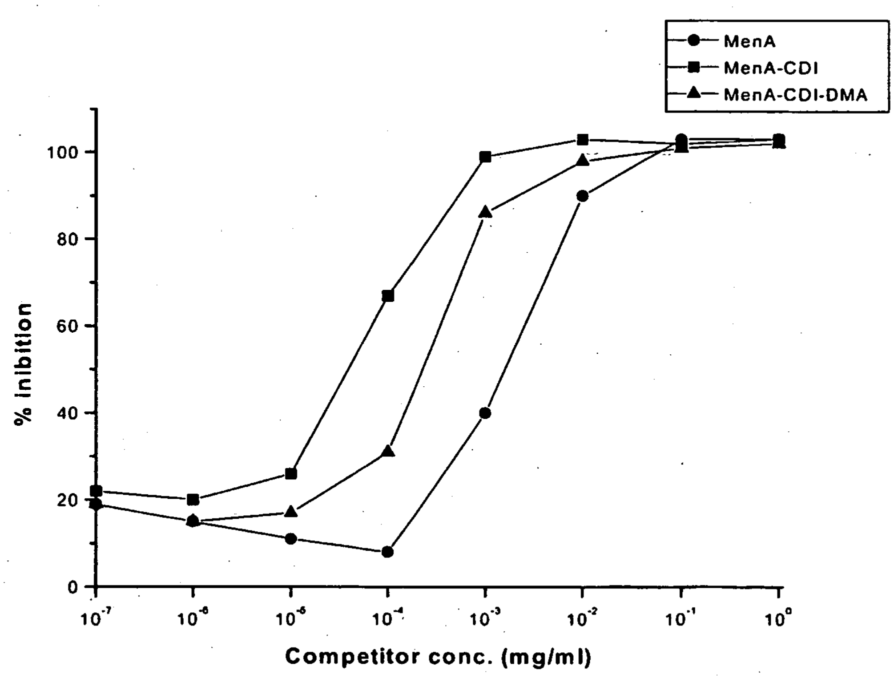

*Figure 8*

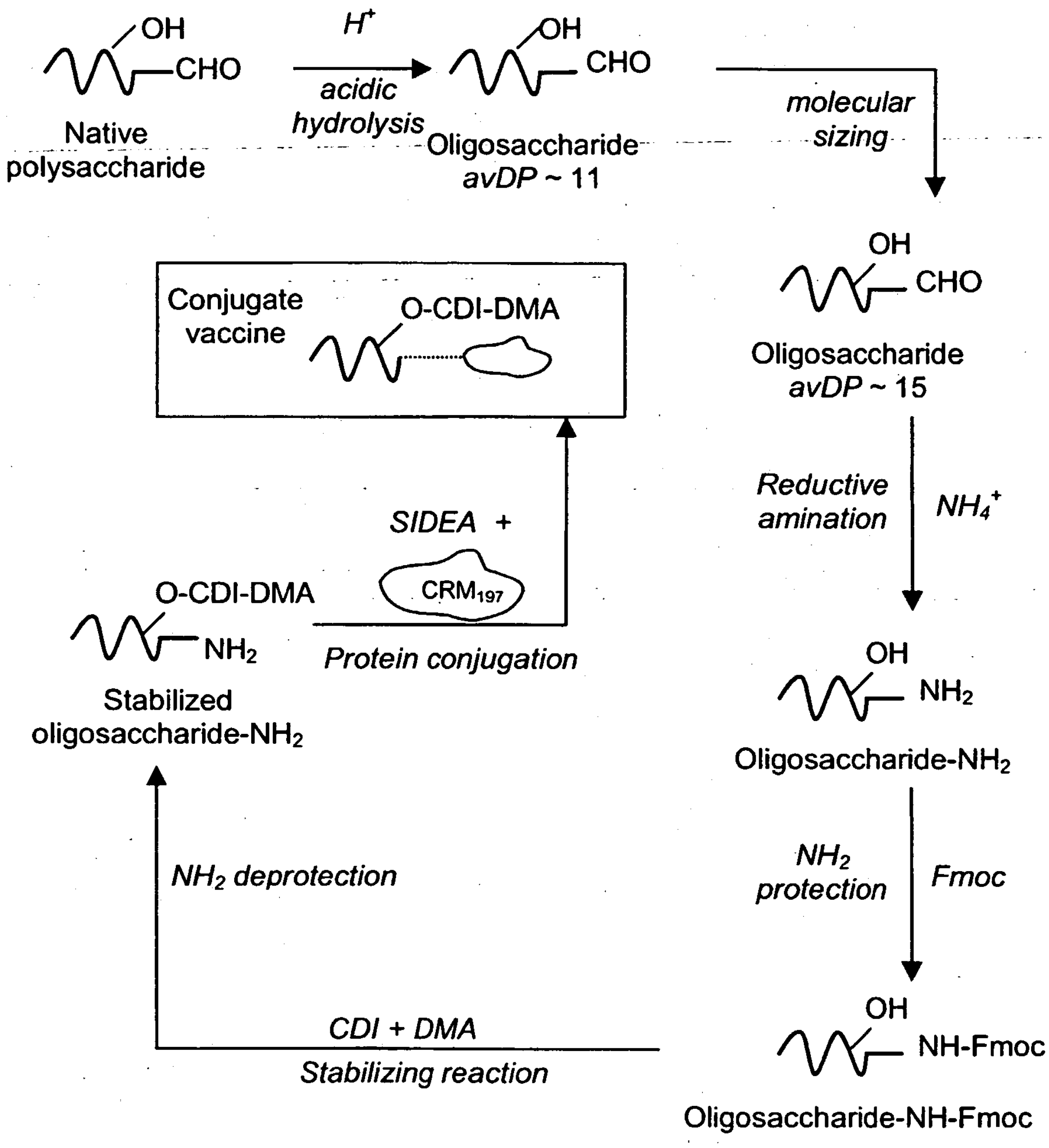
H+
acidic
hydrolysis
Native
polysaccharide
Oligosaccharide
avDP ~ 11
molecular
sizing
Oligosaccharide
avDP ~ 15
Reductive
amination
NH4+
Oligosaccharide-NH2
NH2
protection
Fmoc
Oligosaccharide-NH-Fmoc
CDI + DMA
Stabilizing reaction
NH2 deprotection
Stabilized
oligosaccharide-NH2
SIDEA +
CRM197
Protein conjugation
Conjugate
vaccine

*Figure 9*

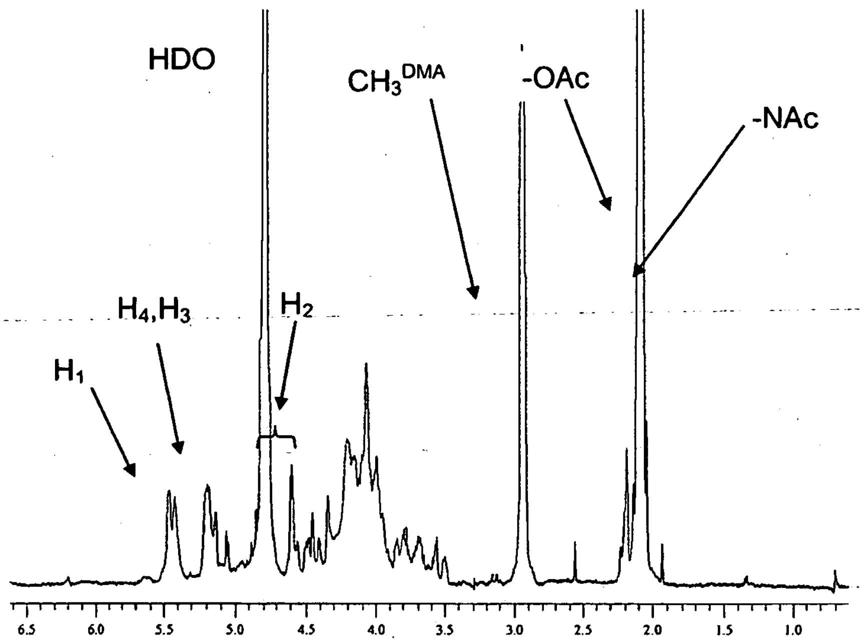


*Figure 11*

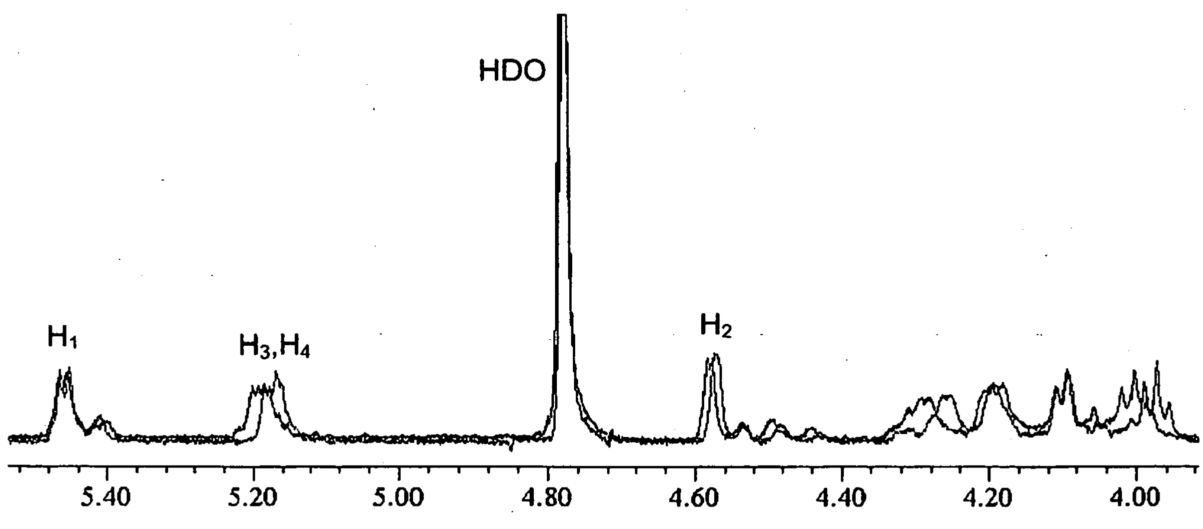

*Figure 10A*

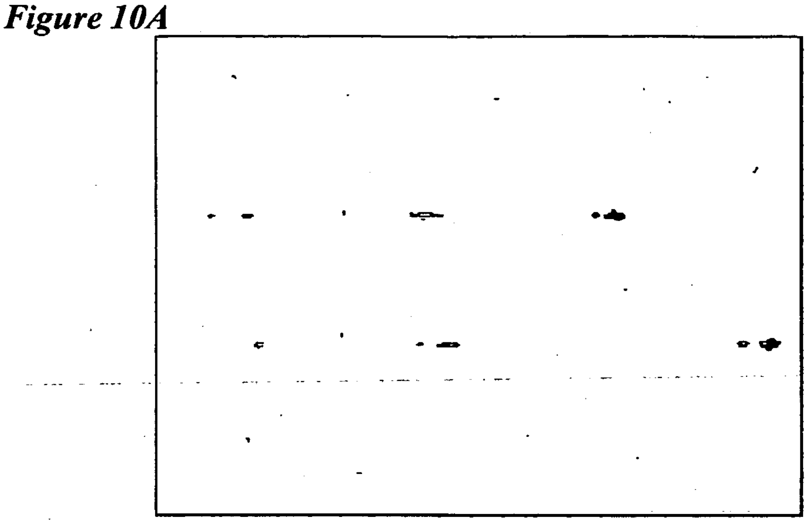

*Figure 10B*

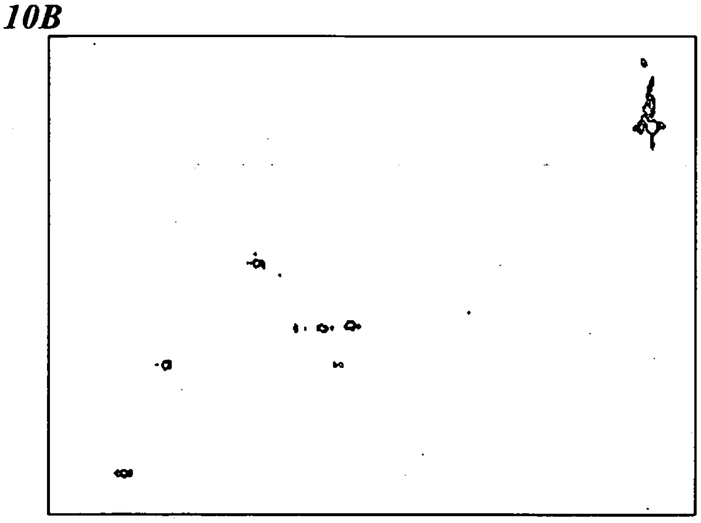

*Figure 12*

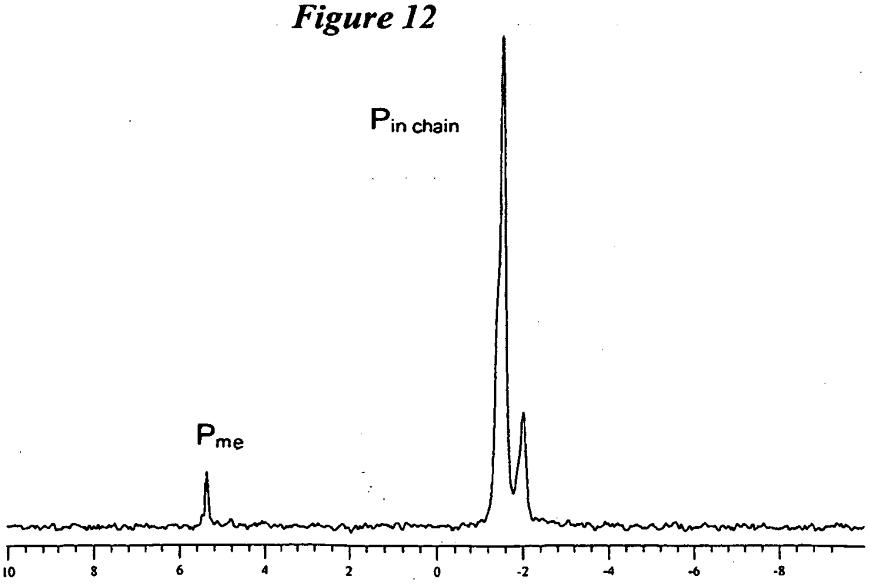

*Figure 13*

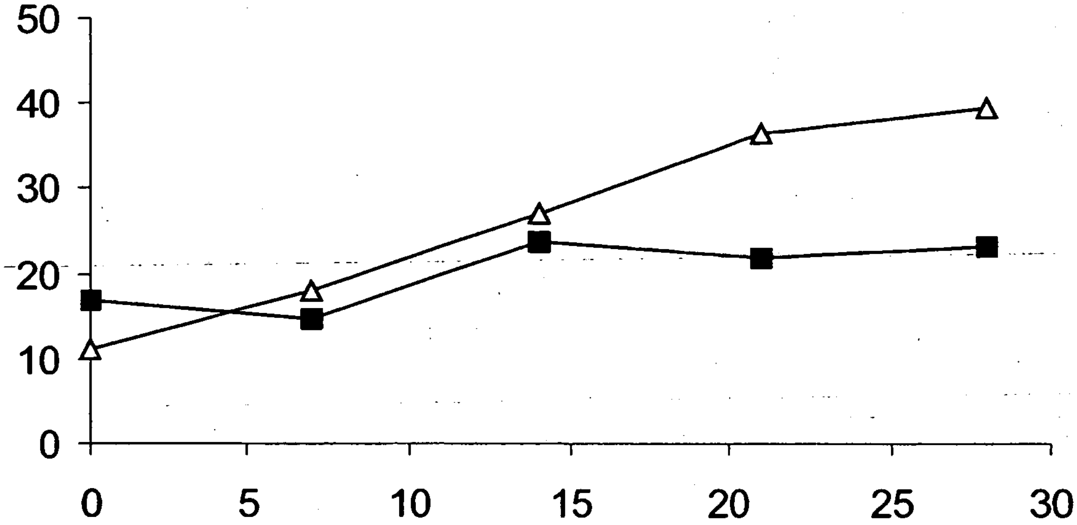


*Figure 14*

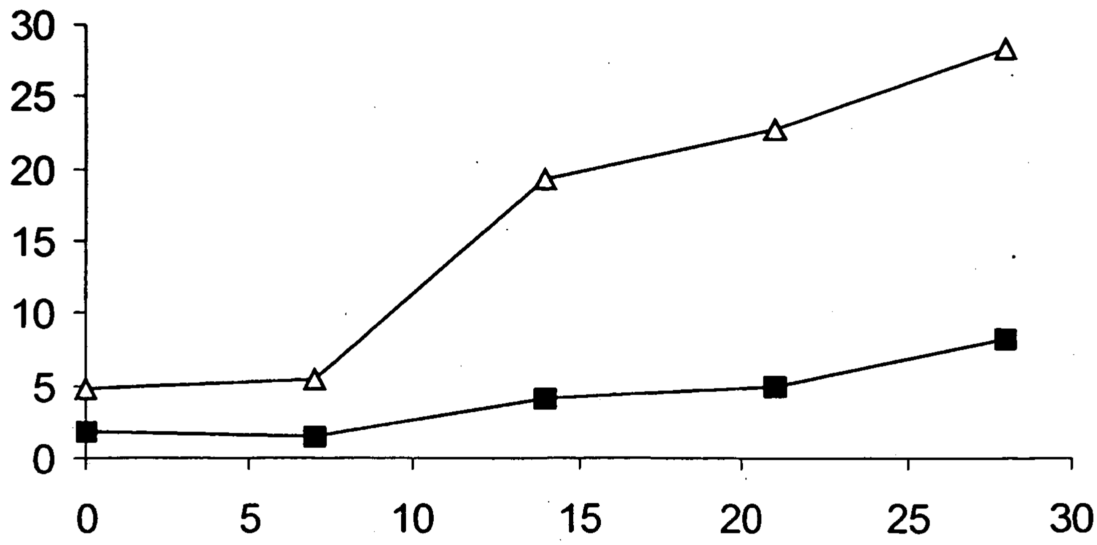

*Figure 15*

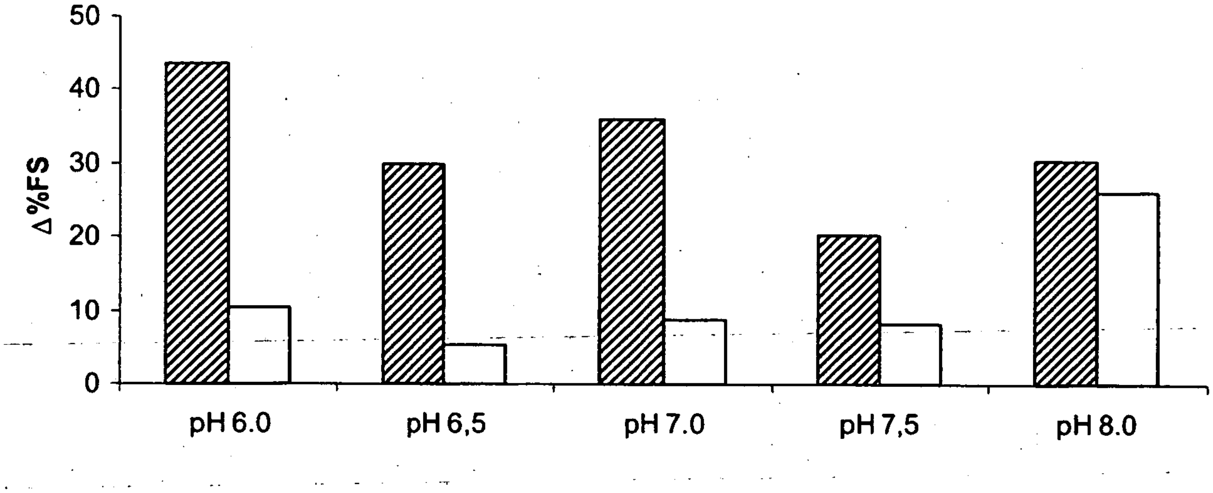


*Figure 16*

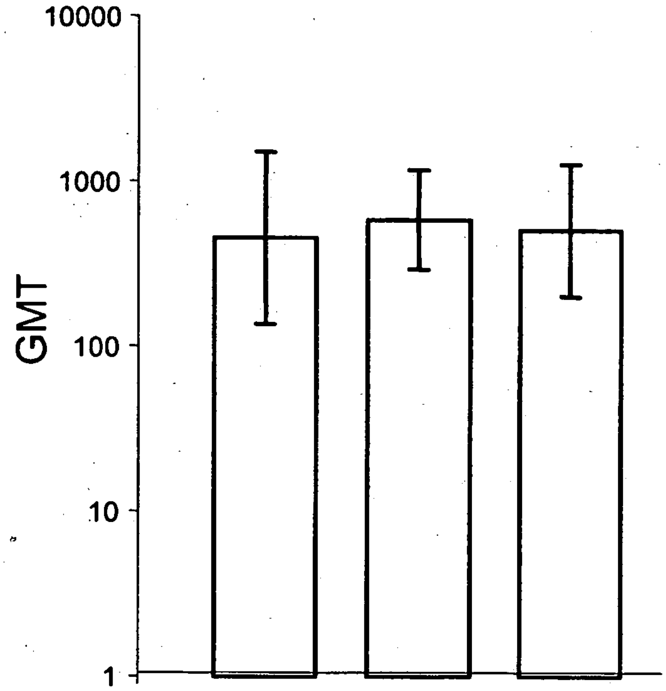

*Figure 17*

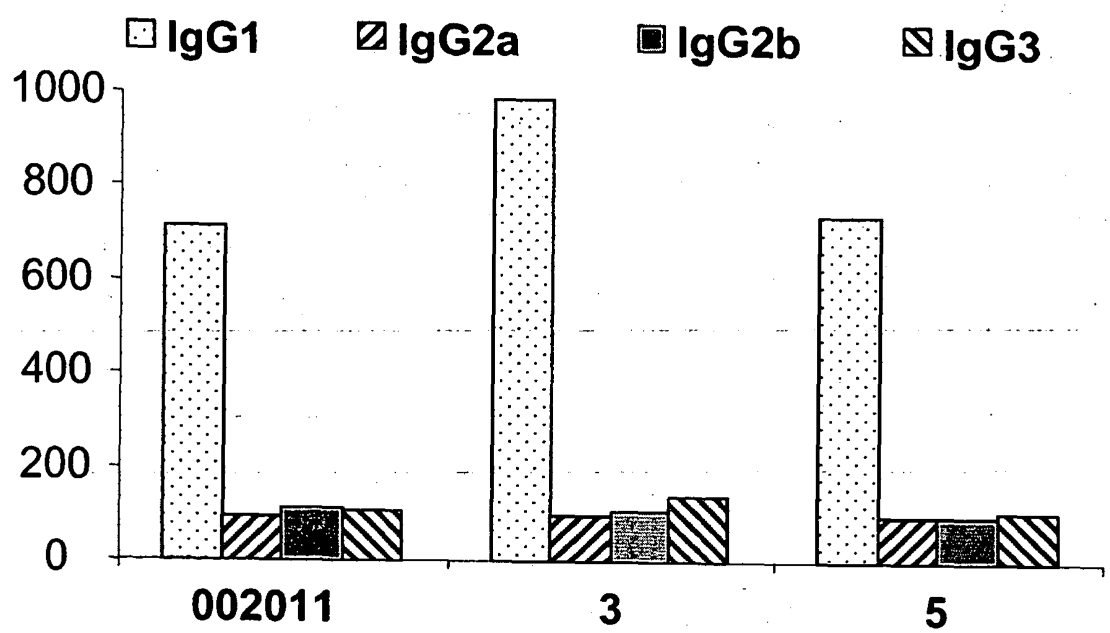


*Figure 18*

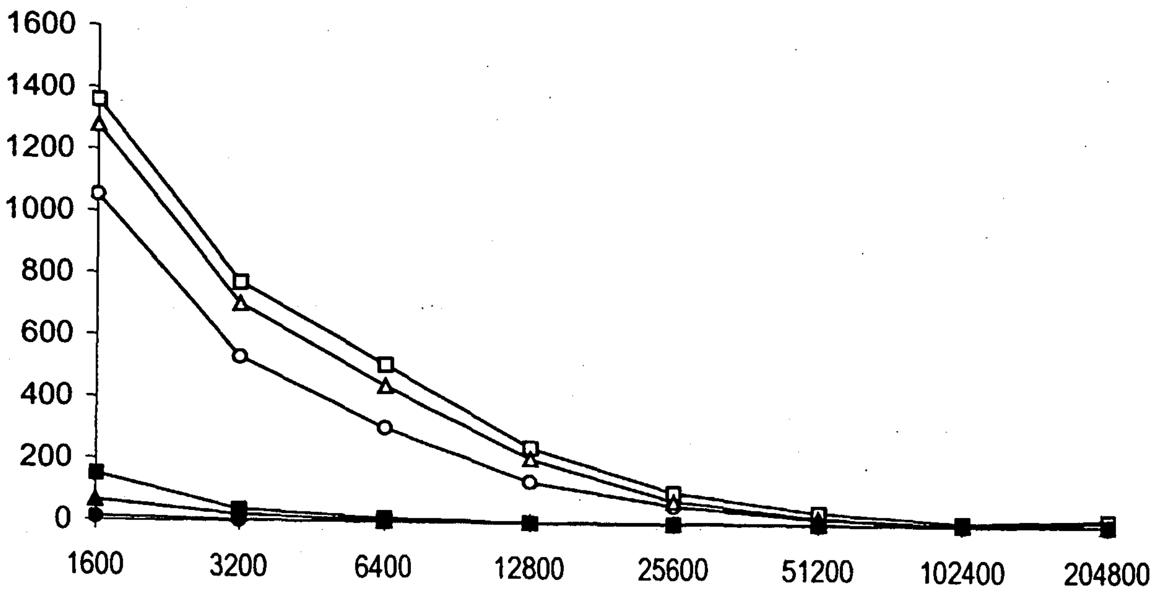

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 733459 A
- WO 9842718 A
- WO 9307178 A
- US 4711779 A
- US 4761283 A
- US 4882317 A
- US 4356170 A
- US 4695624 A
- EP 0477508 B
- US 5306492 A
- WO 9842721 A
- EP 0208375 A
- WO 0010599 A
- US 4057685 A
- US 4673574 A
- US 4808700 A
- US 4459286 A
- US 4965338 A
- US 4663160 A
- EP 0372501 A
- EP 0378881 A
- EP 0427347 A
- WO 9317712 A
- WO 9403208 A
- WO 9858668 A
- EP 0471177 A
- WO 0056360 A
- WO 9101146 A
- WO 0061761 A
- WO 0172337 A
- WO 0038711 A
- WO 9942130 A
- WO 9640242 A
- WO 0056365 A
- WO 9820734 A
- WO 9014837 A
- WO 0007621 A
- WO 9927960 A
- EP 0835318 A
- EP 0735898 A
- EP 0761231 A
- WO 9602555 A
- WO 9816247 A
- WO 9818810 A
- WO 9840100 A
- WO 9855495 A
- WO 9837919 A
- WO 9852581 A
- WO 9952549 A
- WO 0121207 A
- WO 0121152 A
- WO 0062800 A
- WO 0023105 A
- WO 9911241 A
- WO 9857659 A
- WO 0050078 A
- WO 03007985 A
- WO 9318150 A
- WO 9601272 A
- WO 9601273 A
- WO 9725429 A
- WO 9804702 A
- WO 9924578 A
- WO 9936544 A
- WO 9957280 A
- WO 0022430 A
- WO 9629412 A
- WO 0152885 A
- WO 0202606 A
- WO 9927105 A
- WO 0027994 A
- WO 0037494 A
- WO 9928475 A
- WO 0234771 A


### Non-patent literature cited in the description


- **NILSSON ; SVENSSON.** *Carbohydrate Research,* 1979, vol. 69, 292-296
- **RAMSAY et al.** *Lancet,* 2001, vol. 357 (9251), 195-6
- **LINDBERG.** *Vaccine,* 1999, vol. 17 (2), 28-36
- **BUTTERY ; MOXON.** *J R Coll Physicians Lond,* 2000, vol. 34, 163-8
- **AHMAD ; CHAPNICK.** *Infect Dis Clin North Am,* 1999, vol. 13, 113-33
- **GOLDBLATT.** *J. Med. Microbiol.,* 1998, vol. 47, 563-567
- **DICK et al.** Conjugate Vaccines. 1989, vol. 10, 48-114
- **HERMANSON.** Bioconjugate Techniques. Academic Press, 1996

- **BETHELL G.S. et al.** *J. Biol. Chem.,* 1979, vol. 254, 2572-4
- **HEARN M.T.W.** *J. Chromatogr.,* 1981, vol. 218, 509-18
- *Mol. Immunol.,* 1985, vol. 22, 907-919
- **GEVER et al.** *Med. Microbiol. Immunol,* 1979, vol. 165, 171-288
- **LEI et al.** *Dev Biol (Basel),* 2000, vol. 103, 259-264
- Vaccine design: the subunit and adjuvant approach. Plenum Press, 1995
- **KRIEG.** *Vaccine,* 2000, vol. 19, 618-622
- **KRIEG.** *Curr opin Mol Ther,* 2001, vol. 3, 15-24
- **DEL GIUDICE et al.** *Molecular Aspects of Medicine,* 1998, vol. 19
- **JOHNSON et al.** *Bioorg Med Chem Lett,* 1999, vol. 9, 2273-2278
- **SINGH et al.** *J. Cont. Rele.,* 2001, vol. 70, 267-276
- **COVACCI ; RAPPUOLI.** *J. Exp. Med.,* 2000, vol. 19, 587-592
- **COVACCI et al.** *Proc. Natl. Acad. Sci. USA,* 1993, vol. 90, 5791-5795
- **TUMMURU et al.** *Infect. Immun.,* 1994, vol. 61, 1799-1809
- **MARCHETTI et al.** *Vaccine,* 1998, vol. 16, 33-37
- **TELFORD et al.** *J. Exp. Med.,* 1994, vol. 179, 1653-1658
- **EVANS et al.** *Gene,* 1995, vol. 153, 123-127
- **TETTELIN et al.** *Science,* 2000, vol. 287, 1809-1815
- **PIZZA et al.** *Science,* 2000, vol. 287, 1816-1820
- **BJUNE et al.** *Lancet,* 1991, vol. 338 (8775), 1093-1096
- **FUKASAWA et al.** *Vaccine,* 1999, vol. 17, 2951-2958
- **ROSENQVIST et al.** *Dev. Biol. Stand.,* 1998, vol. 92, 323-333
- **COSTANTINO et al.** *Vaccine,* 1992, vol. 10, 691-698
- **COSTANTINO et al.** *Vaccine,* 1999, vol. 17, 1251-1263
- **WATSON.** *Pediatr Infect Dis J,* 2000, vol. 19, 331-332
- **RUBIN.** *Pediatr Clin North Am,* 2000, vol. 47, 269-285
- **JEDRZEJAS.** *Microbiol Mol Biol Rev,* 2001, vol. 65, 187-207
- **BELL.** *Pediatr Infect Dis J,* 2000, vol. 19, 1187-1188
- **IWARSON.** *APMIS,* 1995, vol. 103, 321-326
- **GERLICH et al.** *Vaccine,* 1990, vol. 8, S63-6879-80
- **HSU et al.** *Clin Liver Dis,* 1999, vol. 3, 901-915
- **GUSTAFSSON et al.** *N. Engl. J. Med.,* 1996, vol. 334, 349-355
- **RAPPUOLI et al.** *TIBTECH,* 1991, vol. 9, 232-238
- Vaccines. 1988
- **DEL GUIDICE et al.** *Molecular Aspects of Medicine,* 1998, vol. 19, 1-70
- **KALMAN et al.** *Nature Genetics,* 1999, vol. 21, 385-389
- **READ et al.** *Nucleic Acids Res,* 2000, vol. 28, 1397-406
- **SHIRAI et al.** *J. Infect. Dis.,* 2000, vol. 181, S524-S527
- **ROSS et al.** *Vaccine,* 2001, vol. 19, 4135-4142
- **SUTTER et al.** *Pediatr Clin North Am,* 2000, vol. 47, 287-308
- **ZIMMERMAN ; SPANN.** *Am Fam Physician,* 1999, vol. 59, 113-118125-126
- **DREESEN.** *Vaccine,* 1997, vol. 15, 2-6
- *MMWR Morb Mortal Wkly Rep,* 16 January 1998, vol. 47 (1), 12, 19
- **MCMICHAEL.** *Vaccine,* 2000, vol. 19 (1), 101-107
- **SCHUCHAT.** *Lancet,* 1999, vol. 353 (9146), 51-6
- **DALE.** *Infect Dis Clin North Am,* 1999, vol. 13, 227-43
- **FERRETTI et al.** *PNAS USA,* 2001, vol. 98, 4658-4663
- **KURODA et al.** *Lancet,* 2001, vol. 357 (9264), 1225-12401218-1219
- **J TOXICOL CLIN TOXICOL.** *J Toxicol Clin Toxicol,* 2001, vol. 39, 85-100
- **DEMICHELI et al.** *Vaccine,* 1998, vol. 16, 880-884
- **STEPANOV et al.** *J Biotechnol,* 1996, vol. 44, 155-160
- **INGRAM.** *Trends Neurosci,* 2001, vol. 24, 305-307
- **ROSENBERG.** *Nature,* 2001, vol. 411, 380-384
- **MOINGEON.** *Vaccine,* 2001, vol. 19, 1305-1326
- **ROBINSON ; TORRES.** *Seminars in Immunology,* 1997, vol. 9, 271-283
- **DONNELLY et al.** *Annu Rev Immunol,* 1997, vol. 15, 617-648
- **SCOTT-TAYLOR ; DALGLEISH.** *Expert Opin Investig Drugs,* 2000, vol. 9, 471-480
- **APOSTOLOPOULOS ; PLEBANSKI.** *Curr Opin Mol Ther,* 2000, vol. 2, 441-447
- **ILAN.** *Curr Opin Mol Ther,* 1999, vol. 1, 116-120
- **DUBENSKY et al.** *Mol Med,* 2000, vol. 6, 723-732
- **ROBINSON ; PERTMER.** *Adv Virus Res,* 2000, vol. 55, 1-74

- **DONNELLY et al.** *Am J Respir Crit Care Med,* 2000, vol. 162 (4), 190-193
- **DAVIS.** *Mt. Sinai J. Med.,* 1999, vol. 66, 84-90
- **CHEN et al.** *Anal. Chem.,* 1956, vol. 28, 1756-8
- **ANDERSON et al.** *J. Clin. Invest.,* 1985, vol. 76, 52-9
- **WOLFENDEN R.** *J. Am. Chem. Soc.,* 1988, vol. 120, 6814-5
- **HABBEB et al.** *Anal. Biochem,* 1966, vol. 14, 328-336
- **MIRON ; WILCHEK.** *Anal. Biochem.,* 1982, vol. 126, 433-435
- **RICCI et al.** *Vaccine,* 2001, vol. 19, 1989-1997
- **CARLONE et al.** *J. Clin. Microbiol.,* 1992, vol. 30, 154-159